# EUROPEAN PATENT SPECIFICATION

(11) **EP 3 122 372 B1**
(45) Date of publication and mention of the grant of the patent: **28.11.2018**
(21) Application number: 15727086.9
(22) Date of filing: 26.03.2015
(51) Int. Cl.: A61K 38/46, A61P 31/12

(54) **ONCONASE FOR USE IN TREATING OR PREVENTING VIRAL INFECTIONS**
ONCONASE ZUR BEHANDLUNG ODER VORBEUGUNG VON VIRUSINFEKTIONEN
ONCONASE POUR UNE UTILISATION DANS LE TRAITEMENT OU LA PRÉVENTION D'INFECTIONS VIRALES

(30) Priority: 28.03.2014 US 201414229816; 08.04.2014 US 201414247723; 27.06.2014 US 201414316893; 22.08.2014 US 201462040885 P; 14.10.2014 US 201462063551 P; 13.01.2015 US 201562102671 P; 24.03.2015 US 201514667282
(43) Date of publication of application: 01.02.2017
(73) Proprietor: Tamir Biotechnology, Inc., San Diego, California 92130 (US)
(72) Inventor: HODGE, Thomas, Athens, Georgia 30606 (US)
(74) Representative: Lasar, Andrea Gisela
(86) International application number: PCT/US2015/022670
(87) International publication number: WO 2015/148768

(56) References cited:
- WO-A1-2005/080586
- US-A1- 2012 121 569
- US-A1- 2013 022 589
- "Tamir Reports Positive Effect Against SARS Virus", , 21 October 2010 (2010-10-21), pages 1-3, XP055202166, Retrieved from the Internet: URL:http://globenewswire.com/news-release/ 2010/07/21/425595/197044/en/Tamir-Reports- Positive-Effect-Against-SARS-Virus.html [retrieved on 2015-07-14]
- "Tamir's Compounds Show Remarkable Results Against Dengue Virus", , 19 July 2010 (2010-07-19), pages 1-3, XP055202167, Retrieved from the Internet: URL:http://www.globenewswire.com/news-rele ase/2010/07/19/425341/196757/en/Tamir-s-Co mpounds-Show-Remarkable-Results-Against-De ngue-Virus.html [retrieved on 2015-07-14]
- Anonymous: "Alfacell Corporation - Anti Viral Status in Quarterly Report for Tamir Biotechnology - ACEL - InvestorVillage", , 15 August 2011 (2011-08-15), pages 1-2, XP055202177, Retrieved from the Internet: URL:http://www.investorvillage.com/mbthrea d.asp?mb=470&tid=10843482&showall=1 [retrieved on 2015-07-14]
- D BALTIMORE: "Expression of animal virus genomes", BACTERIOLOGICAL REVIEWS, vol. 35, no. 3, 1 September 1971 (1971-09-01), pages 235-241, XP055202217, UNITED STATES ISSN: 0005-3678
- Anonymous: "Baltimore classification - Wikipedia, the free encyclopedia", , 6 March 2013 (2013-03-06), pages 1-5, XP055202225, Retrieved from the Internet: URL:https://en.wikipedia.org/w/index.php?t itle=Baltimore_classification&oldid=542396 320 [retrieved on 2015-07-14]
- ILINSKAYA O N ET AL: "Ribonucleases as antiviral agents", MOLEKULYARNAYA BIOLOGIYA, XX, XX, vol. 48, no. 5, 11 October 2014 (2014-10-11), pages 615-623, XP035408512, ISSN: 0026-8933, DOI: 10.1134/S0026893314040050 [retrieved on 2014-10-11]
- MENG QIAO ET AL: "Onconase downregulates microRNA expression through targeting microRNA precursors", CELL RESEARCH, vol. 22, no. 7, 24 April 2012 (2012-04-24) , pages 1199-1202, XP055202281, ISSN: 1001-0602, DOI: 10.1038/cr.2012.67
- LIN J J ET AL: "Characterization of the Mechanism of Cellular and Cell Free Protein Synthesis Inhibition by an Anti-tumor Ribonuclease", BIOCHEMICAL AND BIOPHYSICAL RESEARCH COMMUNICATIONS, ACADEMIC PRESS INC. ORLANDO, FL, US, vol. 204, no. 1, 15 October 1994 (1994-10-15), pages 156-162, XP024765866, ISSN: 0006-291X, DOI: 10.1006/BBRC.1994.2439 [retrieved on 1994-10-15] cited in the application
- JOANNE M MOREAU ET AL: "Diminished expression of an antiviral ribonuclease in response to pneumovirus infection in vivo", ANTIVIRAL RESEARCH, vol. 59, no. 3, 1 August 2003 (2003-08-01) , pages 181-191, XP055222280, NL ISSN: 0166-3542, DOI: 10.1016/S0166-3542(03)00111-6
- J. B. DOMACHOWSKE ET AL: "Eosinophil cationic protein/RNase 3 is another RNase A-family ribonuclease with direct antiviral activity", NUCLEIC ACIDS RESEARCH, vol. 26, no. 14, 1 July 1998 (1998-07-01), pages 3358-3363, XP055222291, GB ISSN: 0305-1048, DOI: 10.1093/nar/26.14.3358

## Description

### Field of the Invention

The present disclosure relates to the use of an RNase of the RNase A superfamily and in particular of ranpirnase for the treatment or prevention of viral infection by viruses classified in Baltimore Classification Groups V, IV, II and I in a mammalian subject.

### Background of the Invention

Viral infections may affect different organs such as the lungs and the gastrointestinal tract, causing severe diseases or even the death of the infected subject.

For example, the ongoing Ebola epidemic in West Africa has so far caused the death of about 10,000 people. So far, there is no approved treatment for infections with Ebola virus, although several experimental drugs have been tested.

As another example, influenza virus causes millions of infections every year and is responsible for several thousand deaths worldwide per year. For influenza infections no efficient treatment is available.

A press release by Tamir Biotechnology, Inc. of 21-07-2010 announced that Onconase(R) (Ranpirnase) showed activity against Severe Acute Respiratory Syndrome (SARS) virus.

Hence, there is a need to identify substances which can be used to efficiently treat or prevent viral diseases.

### Summary of the Invention

The invention is defined as set forth in the claims. All other embodiments are described for illustrative purposes only, and any statement that they would also be part of the invention or other, similar statements, have to be seen in the context of the application as filed and do not alter the scope of the claims.

Recent experiments have shown that ranpirnase demonstrates surprisingly strong antiviral effects against a surprisingly large number of different viruses, including viruses (e.g. MERS-CoV and EBOV) that are highly resistant to treatment.

Accordingly, the present disclosure relates to an RNase of the RNase A superfamily or a functional derivative thereof for use in treating or preventing viral infection in a mammalian subject, wherein the virus is classified in Baltimore Classification Group V.

Preferably, the virus classified in Baltimore Classification Group V is from a family selected from the group consisting of rhabdoviridae, paramyxoviridae, orthomyxoviridae and filoviridae and more preferably it is selected from the group consisting of rabies, influenza A, measles, Ebola virus and respiratory syncytial virus.

In another embodiment, the present disclosure relates to an RNase of the RNase A superfamily or a functional derivative thereof for use in treating or preventing viral infection in a mammalian subject, wherein the virus is classified in Baltimore Classification Group IV and is other than dengue virus, yellow fever virus and severe acute respiratory syndrome (SARS) coronavirus.

Preferably, the virus classified in Baltimore Classification Group IV is from a family selected from the group consisting of togaviridae, coronaviridae and picornaviridae and more preferably it is selected from the group consisting of MERS-CoV, Chikungunya, Venezuelan equine encephalitis and rhinovirus-14.

In still another embodiment, the present disclosure relates to an RNase of the RNase A superfamily or a functional derivative thereof for use in treating or preventing viral infection in a mammalian subject, wherein the virus is from a family selected from the group consisting of adenoviridae and poxviridae.

Preferably, the virus is selected from the group consisting of vaccinia virus and adenovirus 2.

In still another embodiment, the present disclosure relates to an RNase of the RNase A superfamily or functional derivative thereof for use in treating or preventing viral infection in a mammalian subject, wherein the virus is classified in Baltimore Classification Group II.

Preferably, the virus classified in Baltimore Classification Group II is from the parvoviridae family and more preferably it is canine parvovirus.

Preferably, the RNase of the RNase A superfamily has an amino acid sequence selected from the group consisting of SEQ ID Nos. 1, 2, 3 and 4 and more preferably, it has the amino acid sequence according to SEQ ID No.1 which is the amino acid sequence of ranpirnase.

In another preferred embodiment the mammalian subject is a human.

Also preferably, the RNase is administered systemically, more preferably it is administered by intravenous, intramuscular, oral, rectal or nasal administration.

Specifically, the present disclosure relates to ranpirnase for use in treating or preventing viral infection in a mammalian subject, wherein the virus is selected from the group consisting of rabies virus, MERS-CoV, influenza virus, Ebola virus, Chikungunya virus, Venezuelan equine encephalitis virus, canine parvovirus, adenovirus-2, respiratory syncytial virus, rhinovirus-14 and vaccinia virus.

### Brief Description of the Drawings

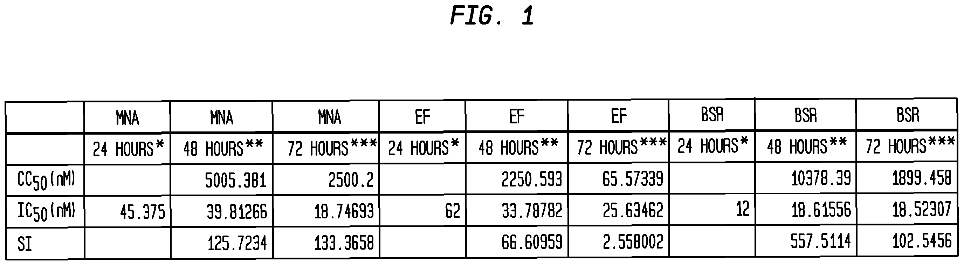

Figure 1 shows the results of testing the anti-viral activity of ranpirnase against rabies virus in mouse neuroblastoma cells ("MNA"), bat primary epithelial cells ("EF") and cloned baby hamster kidney cells ("BSR"). *: after incubation with ranpirnase, before infection with rabies virus; **: 24 hours after infection with rabies virus; ***: 48 hours after infection with rabies virus

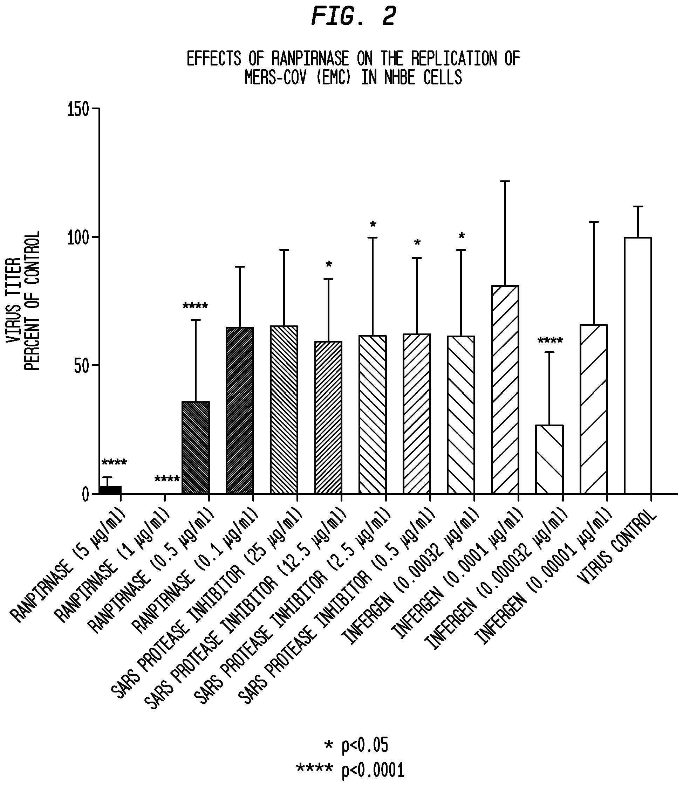

Figure 2 shows the results of testing the anti-viral activity of various concentrations as indicated of ranpirnase against MERS-CoV virus in normal human bronchial epithelial ("NHBE") cells, compared with the anti-viral activities of various concentrations as indicated of SARS protease inhibitor and Infergen; *: p< 0.05; ****: p< 0.0001

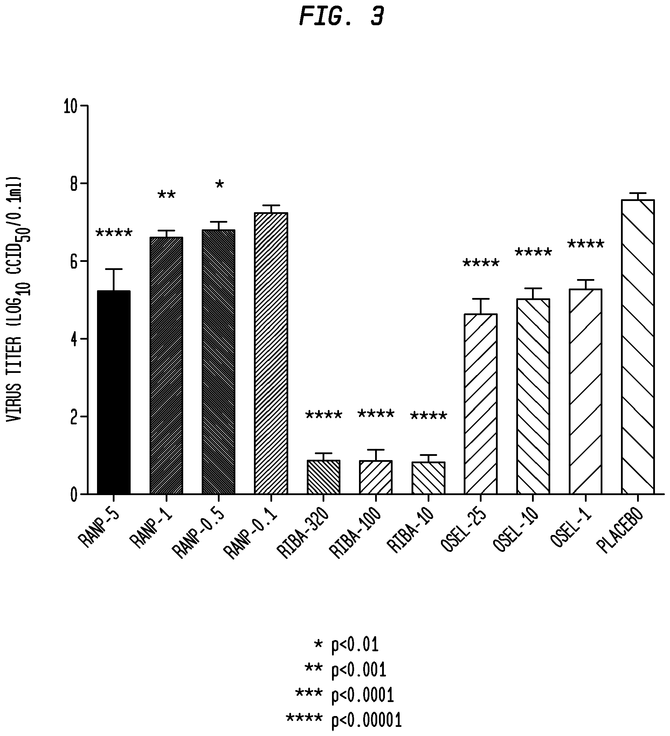

Figure 3 shows the results of testing the anti-viral activity of various concentrations as indicated of ranpirnase ("Ranp") against a strain of influenza in NHBE cells, compared with the anti-viral activities of various concentrations as indicated of Ribavirin ("Riba") and Oseltamivir carboxylate ("Osel"); *: p< 0.01; **: p < 0.001; ***: p< 0.0001; ****: p < 0.00001

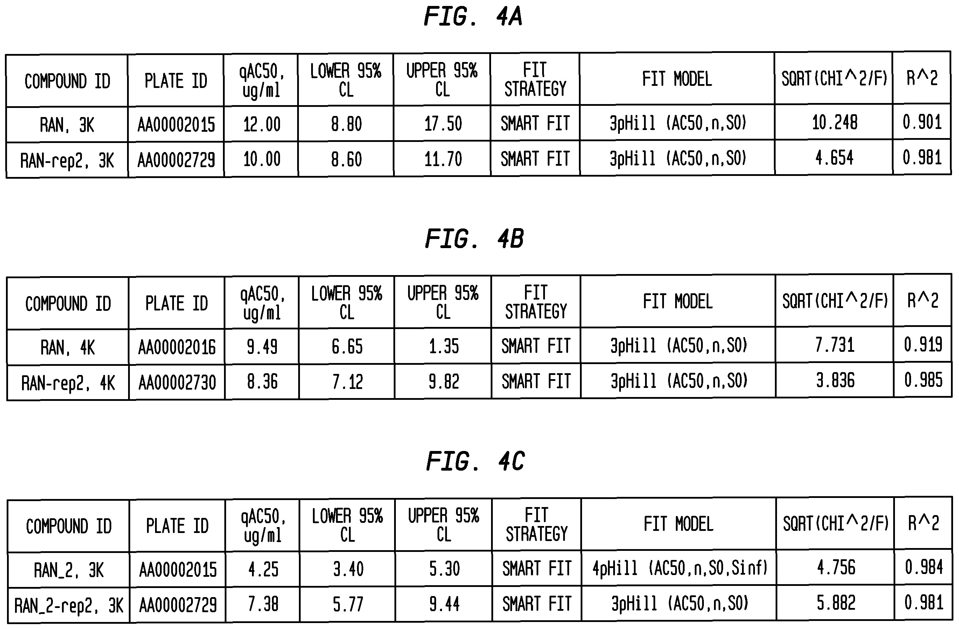

Figure 4 a) shows the effect of ranpirnase (shown as "RAN") against VEEV infection in astrocytes plated at 3000 cells/well (shown as "3k");
Figure 4 b) shows the effect of ranpirnase (shown as "RAN") against VEEV infection in astrocytes plated at 4000 cells/well (shown as "4k");
Figure 4 c) shows the effect of lyophilized ranpirnase powder (shown as "RAN_2") against VEEV infection in astrocytes plated at 3000 cells/well (shown as "3k") ;
Figure 5 shows the effect of ranpirnase against VEEV infection in HeLa cells;
Figure 6 shows the effect of ranpirnase against CHIV infection in U2OS cells;
Figure 7 shows the effect of ranpirnase against EBOV infection in HeLa cells;
Figure 8 shows the effect of ranpirnase against EBOV infection in Vero E6 cells;
Figure 9 shows the AC50 toxicity values for ranpirnase inhibition of VEEV, CHIV, and EBOV;
Figure 10 shows the doses used in a dose response study of ranpirnase in mice infected with EBOV;
Figure 11 shows mouse survival in the study of Figure 10;
Figure 12 is a graphical representation of the data in Figure 11, shown in percentage terms;
Figure 13 shows mouse weight loss in the study of Figure 10;
Figure 14 shows mouse weight loss in the study of Figure 10 in percentage terms;
Figure 15 is a graphical representation of the data in Figure 14;
Figure 16 shows the doses of ranpirnase used in a study of ranpirnase inhibition of AV in NHBE cells;
Figure 17 shows the results of the study of Figure 16;
Figure 18 shows the results of a study of ranpirnase inhibition of canine parvovirus in A-72 cells;
Figure 19 shows the doses of ranpirnase used in a study of ranpirnase inhibition of RSV in NHBE cells;
Figure 20 shows the results of the study of Figure 19;
Figure 21 shows the doses of ranpirnase used in a study of ranpirnase inhibition of RV-14 in NHBE cells;
Figure 22 shows the results of the study of Figure 21;
Figure 23 shows the doses of ranpirnase used in a study of ranpirnase inhibition of vaccinia in Vero 76 cells; and
Figure 24 shows the results of the study of Figure 23.

### DETAILED DESCRIPTION OF THE INVENTION

Recent experiments have shown that ranpirnase demonstrates surprisingly strong antiviral effects against a surprisingly large number of different viruses, including viruses (e.g. MERS-CoV and EBOV) that are highly resistant to treatment.

It is believed that the surprisingly broad-spectrum activity of the invention comes from the ways in which ranpirnase degrades various forms of RNA.

To date, three RNA-degrading mechanisms appear to be relevant to antiviral therapy using ranpirnase.

The first of these mechanisms is degradation of tRNA. Degrading tRNA inside a mammalian cell makes that cell resistant to some viral infections. This is because some viruses replicate by protein synthesis using the ribosome, and protein synthesis cannot occur unless transfer RNAs enter the ribosome to deliver the amino acids needed to synthesize the protein. Thus, a systemic application of an agent that degrades tRNA will prevent or at least substantially impede some viruses from spreading to uninfected cells. If this application occurs before the virus has spread widely enough to endanger the host mammal, the virus will eventually die out.

The second mechanism is degradation of viral double-stranded RNA. Some viruses produce double-stranded RNA as part of their process of proliferation in mammalian cells, and destroying that double-stranded RNA can prevent or at least substantially impede replication of such viruses.

The third mechanism is degradation of microRNA and siRNA. In certain viruses that proliferate using double-stranded RNA, that double-stranded RNA is produced by the interaction of microRNA or siRNA with single-stranded RNA. Destroying the microRNA or siRNA can prevent the formation of the viral double-stranded RNA by which the virus replicates.

RNases of the RNase A superfamily are pyrimidine-specific endonucleases found in high quantity in the pancreas of certain mammals and of some reptiles. They are involved in endonucleolytic cleavage of 3'-phosphomononucleotides and 3'-phosphooligonucleotides ending in C-P or U-P with 2',3'-cyclic phosphate intermediates. Members of this superfamily include ranpirnase and variants thereof, amphinase, r-Amphinase-2, bovine seminal vesicle and brain ribonucleases; kidney non-secretory ribonucleases; liver-type ribonucleases, angiogenin; eosinophil cationic protein, and pancreatic ribonucleases from different species including human and bovine pancreatic ribonucleases.

Ranpirnase is an RNase isolated from oocytes of the leopard frog *Rana pipiens* which is disclosed in U.S. Pat. No. 5,559,212, and is also known as Onconase®. The amino acid sequence of ranpirnase is provided in SEQ ID NO: 1. Ranpirnase has been tested and found to be cytotoxic to cancer cells because of its enzymatic activity against RNA.

A variant of ranpirnase (hereinafter, the "'805 variant") is disclosed in U.S. Pat. No. 5,728,805. The '805 variant is also an RNase, and has likewise been found to be cytotoxic to certain cancer cells. The '805 variant is a close variant of ranpirnase; its amino acid sequence is identical to that of ranpirnase except that it has valine instead of isoleucine at position 11, asparagine instead of aspartic acid at position 20, and arginine instead of serine at position 103 of the ranpirnase amino acid sequence. In some embodiments, the '805 variant is referred to as "Val11, Asn20, Arg103-Ranpirnase". The amino acid sequence of the '805 variant is provided in SEQ ID NO:2.

Amphinase 2 is also an RNase. It is the protein identified as 2325p4 in U.S. Pat. No. 6,239,257 and it too has been found to be cytotoxic to cancer cells. The amino acid sequence of Amphinase 2 is provided in SEQ ID NO: 3.

Recombinant Amphinase 2 ("rAmphinase 2") is similar to Amphinase 2, but has a Met residue at position -1 and lacks glycan moieties that are located in Amphinase 2 at positions 27 and 91. rAmphinase 2 is described in U.S. Pat. No. 7,229,824. The amino acid sequence of rAmphinase 2 is provided in SEQ ID NO: 4.

The term "functionally equivalent thereof' is intended to comprise proteins which differ from naturally occurring RNases by one or more amino acids, but retain RNase activity. For example, the '805 variant discussed above may be considered as a functional derivative of ranpirnase.

Ranpirnase is known to degrade tRNA very effectively (see Lin et al., Biochemical and Biophysical Research Communications 201 (1), 156 - 162 (1994)). Because normal mammalian cells degrade approximately 80% of their tRNA as a natural process, this degradation causes little if any harm to the cells themselves. As a result, except in instances where a viral infection has spread too far to be effectively controlled, a systemic application of ranpirnase, particularly before the viral infection has spread too far, causes the virus to die out without killing the normal cells that the virus infects.

The present inventors have found that ranpirnase is effective against the growth of several viruses, including viruses classified in Baltimore Classification Groups V, IV, I and II.

The Baltimore classification of viruses was proposed by David Baltimore based on the mode of gene replication and expression of the virus (Baltimore (1971) Bacteriological Reviews 35: 235-241). According to this classification the viruses can be grouped into seven different groups:
- Group I: double-stranded DNA viruses;
- Group II: single-stranded DNA viruses;
- Group III: double-stranded RNA viruses;
- Group IV: single-stranded RNA viruses with positive sense RNA;
- Group V: single-stranded RNA viruses with negative sense RNA;
- Group VI: positive-sense single-stranded RNA viruses that replicate through a DNA intermediate;
- Group VII: double-stranded DNA viruses that replicate through a single-stranded RNA intermediate.

The present application provides data showing that ranpirnase is broadly applicable for the treatment of viral infections caused by viruses classified in Baltimore Groups I, II, IV and V and in particular viruses classified in Baltimore Groups IV and V.

In Baltimore Classification Group V single-stranded RNA viruses with negative sense RNA are classified. The RNA of these viruses cannot be used directly by the host cell's translational machinery, but first has to be converted to positive-sense RNA by the action of viral polymerases. Virus families within this group include rhabdoviridae, paramyxoviridae, orthomyxoviridae, filoviridae, bunyaviridae and arenaviridae. Within the scope of the present disclosure the treatment or prevention of viral infections caused by viruses from the rhabdoviridae, paramyxoviridae, orthomyxoviridae or filoviridae family is preferred.

The rhabdoviridae family includes the genera Lyssavirus, Ephemerovirus, Novirhabdovirus and Vesiculovirus. The rabies virus belongs to the genus Lyssavirus. Hence, the present disclosure also relates to ranpirnase for use in the treatment or prevention of viral infections caused by viruses of the genus Lyssavirus and in particular by the rabies virus.

The filoviridae family (filovirus) includes the genera Ebolavirus, Marburgvirus, and Cuevavirus. As used herein, the terms "Ebola," "Ebola virus," and "Ebola viruses" refer to all members of the genus Ebolavirus, which includes, without limitation, Bundibugyo virus (BDBV), Sudan virus (SUDV), Tai Forest virus (TAFV, also known as the Cote d'Ivoire Ebolavirus), Ebola virus (EBOV, previously known as Zaire Ebolavirus), and Reston virus (RESTV). The genus Marburgvirus includes Marburg virus (MARV); and the genus Cuevavirus includes Lloviu virus (LLOV). Hence, the present disclosure also relates to ranpirnase for use in the treatment or prevention of viral infections caused by viruses of the genus Ebolavirus and in particular by the Ebola virus.

The paramyxoviridae family includes the genera Paramyxovirus, Morbillivirus and Pneumovirus. The measles virus belongs to the genus Morbillivirus and the respiratory syncytial virus belongs to the genus Pneumovirus. Hence, the present disclosure also relates to ranpirnase for use in the treatment or prevention of viral infections caused by viruses of the genus Morbillivirus or Pneumovirus and in particular by the measles or respiratory syncytial (RSV) virus.

The orthomyxoviridae family includes the genera influenza A, B and C virus. The present invention also relates to ranpirnase for use in the treatment or prevention of viral infections caused by influenza A virus.

In Baltimore Classification Group IV single-stranded RNA viruses with positive sense RNA are classified. The RNA of these viruses can be used directly by the host cell's translational machinery. Virus families within this group include togaviridae, coronaviridae, picornaviridae, flaviviridae and calicivirus. Within the scope of the present disclosure the treatment or prevention of viral infections caused by viruses from the togaviridae, coronaviridae or picornaviridae family is preferred.

The togaviridae family includes the genera Alphavirus and Rubivirus. The genus Alphavirus comprises several species including, without limitation, Chikungunya virus (CHIV), Venezuelan equine encephalitis virus (VEEV), Sindbis virus, Eastern equine encephalitis virus, Western equine encephalitis virus, Ross River virus, Everglades virus, Mucambo virus, Pixuna virus, Middleburg virus, Aura virus, Whataroa virus, Babanki virus, Kyzylagach virus, Highlands J virus, Fort Morgan virus, Ndumu virus, Buggy Creek virus, Barmah Forest virus, Bebaru virus, Cabassou virus, Getah virus, Mayaro virus, Me Tri virus, Rio Negro virus, Tonate virus, Trocara virus, Una virus, O'nyong'nyong virus, and Semliki Forest virus. The genus Rubivirus includes the species rubella virus. Hence, the present disclosure also relates to ranpirnase for use in the treatment or prevention of viral infections caused by viruses of the genus Alphavirus and in particular by the Chikungunya virus (CHIV) or the Venezuelan equine encephalitis virus (VEEV).

The coronaviridae family includes the genera Coronavirus, Betacoronavirus and Torovirus. The Middle East respiratory syndrome coronavirus (MERS-CoV) belongs to the genus Betacoronavirus. Hence, the present disclosure also relates to ranpirnase for use in the treatment or prevention of viral infections caused by viruses of the genus Betacoronavirus and in particular by the MERS-CoV virus.

The picornaviridae family includes the genera Enterovirus, Rhinovirus, Aphthovirus, Cardiovirus and Hepatitis A. Rhinovirus-14 belongs to the genus Rhinovirus. Hence, the present disclosure also relates to ranpirnase for use in the treatment or prevention of viral infections caused by viruses of the genus Rhinovirus and in particular by the Rhinovirus-14.

In Baltimore Classification Group I double-stranded DNA viruses are classified. The mRNA is transcribed in the usual way from the double-stranded DNA using the host cell's transcriptional machinery. Virus families within this group include Adenoviridae, Poxviridae, Iridoviridae, African-swine-fever virus, Herpesviridae, Papovaviridae and Hepadnaviridae. Within the scope of the present disclosure the treatment or prevention of viral infections caused by viruses from the Adenoviridae or Poxviridae family is preferred.

The adenoviridae family includes the genera Mastadenovirus and Aviadenovirus. Adenovirus-2 belongs to the genus Mastadenovirus. Hence, the present disclosure also relates to ranpirnase for use in the treatment or prevention of viral infections caused by viruses of the genus Mastadenovirus and in particular by Adenovirus-2.

The poxviridae family includes the genera Orthopoxvirus, Avipoxvirus, Capripoxvirus, Leporipoxvirus and Parapoxvirus. The vaccinia virus belongs to the genus Orthopoxvirus. Hence, the present disclosure also relates to ranpirnase for use in the treatment or prevention of viral infections caused by viruses of the genus Orthopoxvirus and in particular by the vaccinia virus.

In Baltimore Classification Group I single-stranded DNA viruses are classified. Virus families within this group include parvoviridae, Anelloviridae and Circoviridae. Within the scope of the present disclosure the treatment or prevention of viral infections caused by viruses from the parvoviridae family is preferred.

The parvoviridae family includes the genera Parvovirus and Dependovirus. The canine parvovirus belongs to the genus Parvovirus. Hence, the present disclosure also relates to ranpirnase for use in the treatment or prevention of viral infections caused by viruses of the genus Parvovirus and in particular by canine parvovirus.

In one aspect, the present disclosure relates to an Rnase of the Rnase A superfamily for use in inhibiting the growth or replication of a virus, or for reducing the ability of a virus to infect cells, comprising contacting cells or tissues with said RNase.

The anti-viral effect of each tested compound may be assessed as a 50% effective concentration (EC50) value, which is the concentration at which the amount of viral transcript is reduced by 50% when compared to cultures infected only with the virus and not treated with an RNase. The term EC50 is used interchangeably herein with AC50 (50% active concentration).

The cytotoxic effect of each tested compound is assessed as a 50% cytotoxic concentration (CC50), which is the concentration that results in the death of 50% of the host cells, as measured in a standard cytoxicity assay such as, for example, trypan blue exclusion or mitochondrial function (e.g., MTT) assay.

The National Institute of Allergy and Infectious Diseases (NIAID) (a component of the National Institute of Health) uses a Selectivity Index (SI) ratio as a common indicator to assess the potency of a test compound. The SI, which equals CC50/EC50 (or CC50/AC50), measures the ability of the tested RNase to inhibit replication of a viral infection without killing the infected cells. Where the SI is greater than 1, the RNase under test is active against the virus indicated, and increasing values of SI indicate increasing activity. Because SI measures the ability of a substance under test to inhibit replication of a particular virus without killing the infected cells themselves, it is reasonably correlated with usefulness of the substance in treating a living subject that is infected with the virus. Accordingly, test results in which SI>1 indicate that living subjects infected with the virus tested can be treated by administration of an appropriate dose of the corresponding RNase.

In some aspects, the lower range of SI of the ranpirnase is about 1.0, about 1.5, about 2.0, about 2.5, about 3.0, about 4.0, about 5.0, about 6.0, about 7.0, about 8.0, about 9.0, about 10, about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, about 300, about 325, about 350, about 375, about 400, about 450, about 500, about 550 or about 600.

In some aspects, the upper range of SI of the ranpirnase is about 2.0, about 2.5, about 3.0, about 4.0, about 5.0, about 6.0, about 7.0, about 8.0, about 9.0, about 10, about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, about 300, about 325, about 350, about 375, about 400, about 450, about 500, about 550 or about 600.

In particular aspects, the SI of the ranpirnase ranges from about 1.0 to about 600, about 2.0 to about 450, about 3.0 to about 400, about 4.0 to about 300, about 5.0 to about 250, about 10 to about 100, about 30 to about 100, about 30 to about 50, or about 40 to about 80.

As used herein, the term "about" refers to an amount or number that one of skill in the art would understand is close to the stated amount or number. For example, the term "about" herein refers to an amount that is within 10% above or below the stated amount or number.

The terms "treating" and "treatment," as used herein, refer to administering to a subject infected with a virus a therapeutically effective amount of an RNase such as ranpirnase, a ranpirnase variant such as the '805 variant, Amphinase 2, or rAmphinase 2. As used herein, the term "treating" covers any treatment of a viral disease which results in a desired pharmacologic and/or physiologic effect, including arresting disease development, causing regression of the disease, limiting spread of the virus from one cell to another within an individual, limiting replication of a virus in an individual, limiting entry of a virus into the cell of an individual and reducing the number of viruses in an individual or a tissue of this individual.

The terms "prevention" and "preventing", as used herein, refer to preventing the disease or a symptom thereof from occuring in a subject which is at risk of developing the disease or symptom, but has not yet been diagnosed as having it.

The term "therapeutically effective amount" is used interchangeably herein with the term "therapeutically effective dose" and refers to an amount of an RNase that results in an improvement or remediation of the symptoms of a disease or condition to be treated. A therapeutically effective amount of an RNase such as ranpirnase, '805 variant, Amphinase 2, or rAmphinase 2, in one embodiment, delays or minimizes the onset of, or hastens or increases recovery of a subject from, a virus infection in a subject. In another embodiment, an RNase such as ranpirnase, '805 variant, Amphinase 2, or rAmphinase 2 reduces the overall mortality rate of a disease mediated by a virus infection. In one embodiment, the RNase reduces the viral titer of the infected subject. In another embodiment, the RNase prevents the viral titer of the infected subject from increasing. In one embodiment, a therapeutically effective amount of an RNase provides a therapeutic benefit in the treatment or management of a virus infection or virus-mediated disease. In one embodiment, a therapeutically effective amount of an RNase reduces the spread of the virus from one cell to another. In one embodiment, a therapeutically effective amount of an RNase reduces morbidity or mortality. A therapeutically effective amount may also prevent disease and/or reduce the severity of symptoms.

A therapeutically effective amount can be determined by the skilled person as a matter of routine experimentation. The therapeutically effective dosage of the pharmaceutical composition can be determined readily by the skilled artisan, for example, from animal studies. In addition, human clinical studies can be performed to determine the preferred effective dose for humans by a skilled artisan. Such clinical studies are routine and well known in the art. The precise dose to be employed will also depend on the route of administration. Effective doses may be extrapolated from dose-response curves derived from *in vitro* or animal test systems. The Rnase may be administered to a subject in need thereof in a single dose or in multiple doses. In one embodiment, the RNase is administered to a subject in need thereof once per day, or in multiple doses per day. In one embodiment, the RNase is administered to the subject until symptoms resolve and/or until the subject is no longer at risk of a virus infection.

In some embodiments, the administration of a therapeutically effective amount of the RNase, in particular ranpirnase, reduces the virus titer compared to a control not treated with the RNase, but infected with the virus by at least 10%, preferably by at least 15%, ore preferably by at least 20% and most preferably by at least 25%. Determination of virus titers is discussed in Reischl, Front Biosci. 1996 Aug 1, 1:e 72-7, Application of molecular biology-based methods to the diagnosis of infectious diseases.

In some embodiments, the administration of a therapeutically effective amount of the RNase, in particular ranpirnase, leads to a reduction of the virus titer below the detection level.

In one embodiment, a therapeutically effective dose may be based on the body weight of the subject in need thereof. In one embodiment, a therapeutically effective dose may be in the range of about 0.001 mg/kg to about 1 mg/kg, or about 0.004 to about 0.5 mg/kg, or about 0.02 to about 0.1 mg/kg. In one embodiment, a therapeutically effective dose may be about 0.02 mg/kg, about 0.05 mg/kg, about 0.1 mg/kg, about 0.2 mg/kg, or about 0.5 mg/kg. However, it is apparent that the dosage may vary depending on the compound, the disease and its severity, as well as the age and the weight of the subject to be treated.

In some embodiments, the RNase, in particular ranpirnase, may be administered within 48 hours after exposure to virus. In other embodiments, the RNase may be administered within 72 hours, four days, five, days, six days, seven days, ten days, 14 days, three weeks, four weeks or two months after exposure to virus.

In some embodiments, multiple doses of the RNase, in particular ranpirnase, are administered. The frequency of administration of these multiple doses may vary, depending on factors such as the severity of symptoms. For example, the RNase may be administered once per month, twice per month, every other week, once per week, twice per week, three times per week, four times per week, every other day, once per day, twice per day or three times a day.

The duration of the administration of the RNase, in particular ranpirnase, i.e. the period over which the RNase is administered, can vary depending on factors such as the severity of symptoms, patient response, etc. For example, the RNase can be administered over a period ranging from one day, three days, seven days, two weeks, four weeks, two months, three months, four months, five months or six months or longer.

As used herein, the term "subject" or "patient" refers to any mammal, including, without limitation, humans and other primates, including non-human primates such as chimpanzees and other apes and monkey species. Farm animals such as cattle, sheep, pigs, goats and horses; domestic mammals such as dogs and cats; laboratory animals including rodents such as mice, rats (including cotton rats) and guinea pigs; birds, including domestic, wild and game birds such as chickens, turkeys and other gallinaceous birds, ducks, geese, and the like are also non-limiting examples. Both adult and newborn individuals are intended to be covered.

In particular, the present disclosure relates to the treatment or prevention of virus infections in human subjects. If the viral infection to be treated is an infection with canine parvovirus, the subject is preferably a dog. If the viral infection to be treated is an infection with Venezuelan Equine Encephalitis Virus (VEEV) the subject is preferably a horse, donkey or zebra.

The term "systemic administration" is intended to comprise any route of administration of a medication, nutrition or other substance such that it enters into the circulatory system so that the entire body is affected by the administration. In contrast, topical administration acts only locally at the site of administration.

Generally, a person of ordinary skill in this art would conclude that any route by which the RNase, in particular ranpirnase, is systemically administered will be adequate to treat virus infection (although one route may be more effective than another in any particular instance). Thus, enteral administration (including without limitation oral administration and rectal administration) and parenteral administration (including without limitation intravenous administration, intramuscular administration, and aerosol delivery) are appropriate methods for administration of the RNase, in particular ranpirnase.

Additional exemplary appropriate methods for administration of ranpirnase include nasal, buccal, vaginal, ophthalmic, subcutaneous, intraperitoneal, intraarterial, spinal, intrathecal, intra-articular, intra-arterial, sub-arachnoid, sublingual, oral mucosal, bronchial, lymphatic, intra-uterine, integrated on an implantable device such as a suture or in an implantable device such as an implantable polymer, intradural, intracortical, or dermal. Such compositions would normally be administered as pharmaceutically acceptable compositions as described herein.

The term "pharmaceutical composition" as used herein encompasses a composition suitable for administration to a subject, such as a mammal, especially a human. In general a "pharmaceutical composition" is sterile, and free of contaminants that are capable of eliciting an undesirable response within the subject

The term "pharmaceutically acceptable carrier" as used herein includes any and all solvents, dispersion media, coatings, antibacterial and antifungal agents, isotonic and absorption delaying agents and the like. The use of such media and agents for pharmaceutically active substances is well known in the art. These agents are generally safe, non-toxic and neither biologically nor otherwise undesirable. Except insofar as any conventional media or agent is incompatible with the vectors or cells of the present disclosure its use in therapeutic compositions is contemplated. Supplementary active ingredients also can be incorporated into the compositions. The RNase, in particular ranpirnase and ranpirnase variants provided herein may be administered together with other biologically active agents.

A wide variety of pharmaceutically acceptable excipients is known in the art and therefore is not discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A. H. Kibbe et al., eds., 3rd ed. Amer. Pharmaceutical Assoc.

The RNase, in particular ranoirnase, may be administered to the subject using any convenient means capable of resulting in the desired reduction in viral titers, symptoms of viral infection, etc. Thus, the RNase can be incorporated into a variety of formulations for therapeutic administration. More particularly, the RNase can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, solutions, suppositories, injections, inhalants and aerosols.

Aspects of the disclosure are further illustrated by the following examples that should not be construed as limiting.

### Examples

### Comparative Example 1: Rabies in MEM, EF, and BSR Cells

In the experiments described in Fig. 1, the density of each of the cell lines under test was adjusted to 50,000 cells/ml using minimal essential media (MEM). The cells were placed in wells and incubated at 37 °C and 0.5% CO₂ for 24 hours. Ranpirnase was diluted to concentrations of 10 µM, 3 µM, 900 nM, 270 nM, 81 nM, and 24 nM, added to the wells, and incubated with the cells at 37 °C and 0.5% CO₂ for 24 hours.

Rabies virus at a multiplicity of infection ("MOI") of 0.1 was then added to each of the wells and the ranpirnase and rabies virus - containing wells were incubated at 37 °C and 0.5% CO₂ for 72 hours.

The virus in each of the wells was titrated 24 and 48 hours after introduction of the rabies virus using mouse neuroblastoma cells, and the results are shown in Fig. 1.

As can be seen in Fig. 1 at 24 hours after introduction of viruses into cells treated with ranpirnase, ranpirnase is extraordinarily active against rabies in the mammalian cell lines indicated.

Because SI measures the ability of a substance under test to inhibit replication of a particular virus without killing the infected cells themselves, it is reasonably correlated with usefulness of the substance in treating a mammalian subject that is infected with the virus. Accordingly, test results in which SI > 1 indicate that mammalian subjects infected with rabies can be treated by systemic administration of an appropriate dose of ranpirnase. Furthermore, other below-disclosed experimental results in VEEV, CHIV, and EBOV indicate that it should be possible to use ranpirnase as a prophylactic to prevent rabies infection.

### Comparative Example 2: MERS-CoV in NHBE Cells

In the experiment illustrated in Fig. 2, the anti-viral activity of ranpirnase against MERS-CoV virus was compared to the activities of two known anti-viral agents: SARS protease inhibitor and Infergen. The experiment was carried out using four different concentrations of each agent on normal human bronchial epithelial (NHBE) cells.

More specifically, the NHBE cells were grown in HEPES Buffered Saline Solution at 37 °C for seven days. The cells were washed and refreshed once daily. Two controls were used: one contained MERS-CoV virus and the other contained uninfected NHBE cells that were treated with the test agents.

On the eighth day, the tested concentrations of the three agents under test were introduced into the cells and buffer solution and the virus was introduced at a multiplicity of infection ("MOI") of 0.01 . The virus- and agent-containing samples were then incubated for 72 hours at 37 °C and 5% CO₂, with the medium being replenished once each day. After 72 hours, the samples were then titrated to determine their viral content.

The anti-viral activity of the various agents under test was determined by comparing viral production (viral titer in Vero 76 cells) in NHBE cells that had been treated with the various test agents to the viral production in the NHBE cells used as controls.

As can be seen in Fig. 2, ranpirnase was far more active against MERS-CoV virus than either of the other agents. Further, the activity of ranpirnase was clearly statistically significant, since all but the least concentrated of the doses of ranpirnase had a p value of less than 0.0001.

Because ranpirnase was so effective at inhibiting replication of the MERS-CoV virus in NHBE cells while not killing the host cells, this experiment evidences the likelihood that systemically administered ranpirnase will be useful in treating a mammalian subject infected with a virus, and particularly a mammalian subject infected with MERS-CoV virus. Furthermore, other below-disclosed experimental results in VEEV, CHIV, and EBOV indicate that it should be possible to use ranpirnase as a prophylactic to prevent MERS-CoV infection.

### Example 3: Influenza in NHBE Cells

In the experiment illustrated in Fig. 3, the anti-viral activity of various concentrations (5 µg/ml "Ranp-5", 1 µg/ml "Ranp-1", 0.5 µg/ml "Ranp-0.5", and 0.1 µg/ml "Ranp-0.5") of ranpirnase against influenza A/California/07/2009(H1N1)pdm09 virus was compared to the activities of various concentrations of two known anti-viral agents (Ribavirin at 320 µg/ml "Riba-320", 100 µg/ml "Riba-100", and 10 µg/ml "Riba-20"and Oseltamivir carboxylate at 25 µM "Osel-25", 10 µM "Osel-10", and 1 µM "Osel-1") on NHBE cells. Three controls were used. The first control was NHBE cells that were infected by the virus and treated with a placebo. (This control is shown in Fig. 3.) The second control was NHBE cells that were "infected" with a placebo and treated using the agents under test. The third control was NHBE cells that were "infected" with a placebo and treated with a placebo.

The NHBE cells were supplied with the vendor's proprietary culture medium and equilibrated at 37 °C and 5% CO₂ for at least 16 hours. After equilibration, the cells were washed and refreshed.

NHBE cells were then infected with influenza A/Cahforma/07/2009(H1N1)pdm09 virus at a multiplicity of infection level of 0.01. After an adsorption period of 1 hour, the viral inoculum was removed and the treatments were applied. Twenty four hours post infection, the treatments were replenished. Forty eight hours post infection, the supernatants were harvested. Then, virus titers were determined in Madin-Darby-Canine-Kidney cells and analyzed for statistical significance using one-way analysis of variance.

As can be seen in Fig. 3, with the exception of the least concentrated dose, the activity of ranpirnase against influenza A/California/07/2009(H1N1)pdm09 virus was statistically significant and dose-dependent. Further, these experiments confirmed a result that was seen in the experiment illustrated in Fig. 2: ranpirnase did not decrease the viability of NHBE cells at any of the concentrations under test.

Because ranpirnase inhibited replication of the tested influenza virus in NHBE cells while not killing the cells themselves, this experiment further evidences the likelihood that systemically administered ranpirnase will be useful in treating a mammalian subject infected with a virus, and particularly a mammalian subject infected with influenza. Furthermore, other below-disclosed experimental results in VEEV, CHIV, and EBOV indicate that it should be possible to use ranpirnase as a prophylactic to prevent influenza infection.

### Comparative Example 4: VEEV, CHIV, and EBOV (all in vitro)

### Methodology

Several studies were conducted to assess the ability of ranpirnase to inhibit infection of cells by VEEV, CHIV, and EBOV. Ranpirnase solution (RAN) and powder-derived ranpirnase (RAN-2) were tested. The powder-derived ranpirnase was lyophilized ranpirnase provided by Tamir Biotechnology, Inc. Quality control of the assay was conducted using Positive (Neutral) control (n=16) or infected cells + media, uninfected cells (Negative control) (n=16) and dose response for control inhibitors (n=2 or 4). Z' was calculated for Neutral control and uninfected cells. Data were normalized on the plate bases. Data analysis was done using GeneData software and analysis of dose response curve to determine ED50 of ranpirnase was performed using GeneDataCondoseo software applying Levenberg-Marquardt algorithm (LMA) for curve fitting strategy.

### VEEV in Astrocytes

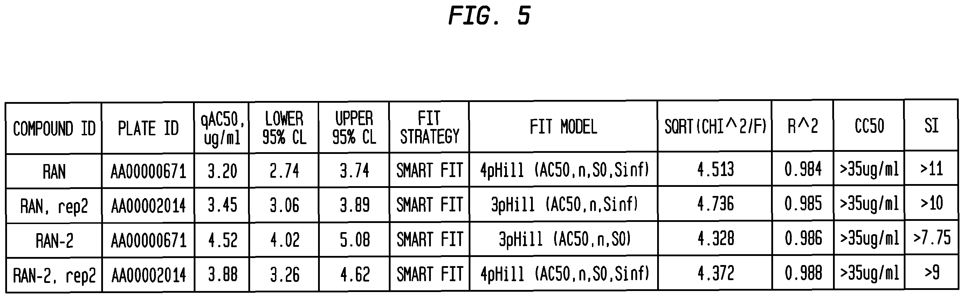

To test the effect of ranpirnase on VEEV infection of astrocytes, ranpirnase solution ("RAN") was tested in duplicated 10 point dose response, and powder-derived ranpirnase ("RAN_2" in Figure 4, "RAN-2" in Figure 5) was re-suspended in phosphate buffered saline at 3.5 mg/ml and was tested only as a single dose response. Both the RAN and the RAN-2 were tested in two independent experiments. In these experiments, astrocytes were plated at 4,000 and 3,000 cells/well, incubated overnight and pre-treated with ranpirnase for 2 hours before the infection. Cells were infected at a multiplicity of infection ("MOI") equal to 0.05 for 20 hours. The results of the study are provided in Figure 4.

To test the effect of ranpirnase on VEEV infection of HeLa cells, RAN was tested in quadruplicated (n=4) 10 point dose response repeated in two independent experiments (rep1 and rep2). RAN-2 was tested in n=2 dose responses on plate and repeated in 2 independent experiments. HeLa cells were plated at 4,000 cells/well, incubated overnight and pre-treated with ranpirnase 2 hours before infection. Cells were infected at an MOI equal to 0.05 for 20 hours. The results of the study are provided in Figure 5. As shown in Figure 5, SI values were over 10 for RAN and over 7.75 for RAN-2.

### CHIV in U2OS Cells

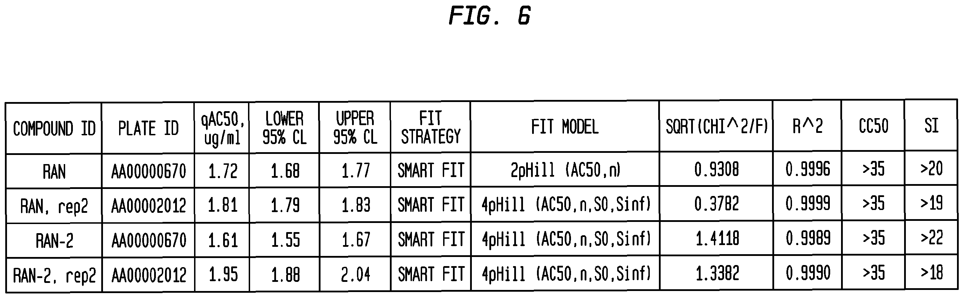

To test the effect of ranpirnase on CHIV infection of U2OS cells, ranpirnase solution was tested in quadruplicated (n=4) 10 point dose response repeated in two independent experiments (rep1 and rep2). The RAN-2 stock was tested in n=2 dose responses on plate and repeated in two independent experiments. U2OS cells were plated at 3,000 cells/well, incubated overnight and pre-treated with ranpirnase 2 hours before infection. Cells were infected at a MOI equal to 0.4 for 24 hours. The results of the study are provided in Figure 6. As shown in Figure 6, SI values were over 18.

### EBOV in HeLa and Vero E6 Cells

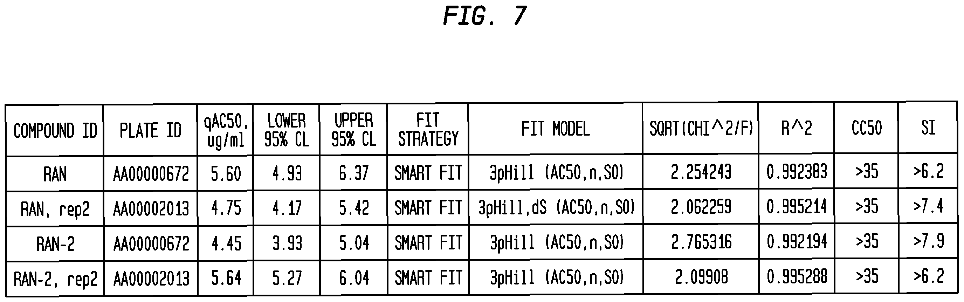

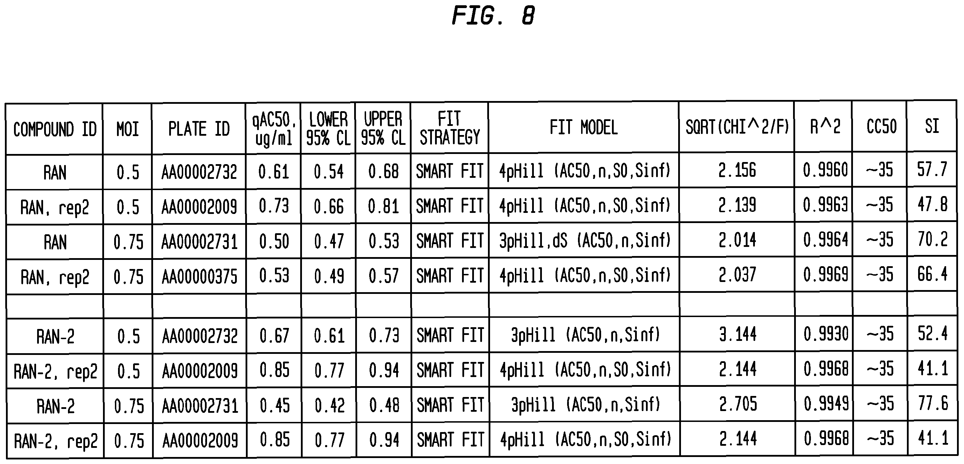

To test the effect of ranpirnase on EBOV infection of HeLa and Vero E6 cells, ranpirnase solution was tested in quadruplicated (n=4) 10 point dose response repeated in two independent experiments (rep1 and rep2). The RAN-2 stock was tested in n=2 dose responses on plate and repeated in two independent experiments. HeLa cells were plated at 4,000 cells/well and Vero E6 at 4000 cells/well, incubated overnight and pre-treated with ranpirnase 2 hours before infection. HeLa cells were infected at a MOI equal to 0.5 and Vero E6 cells were infected at a MOI equal to 0.5 and 0.75 for 48 hours. The results of the study are provided in Figure 7 (HeLa cells) and in Figure 8 (Vero E6 cells). SI values were unexpectedly high, ranging from over 40 to over 77 in Vero E6 cells (Figure 8).

### Summary of in vitro VEEV, CHIV, and EBOV Experiments

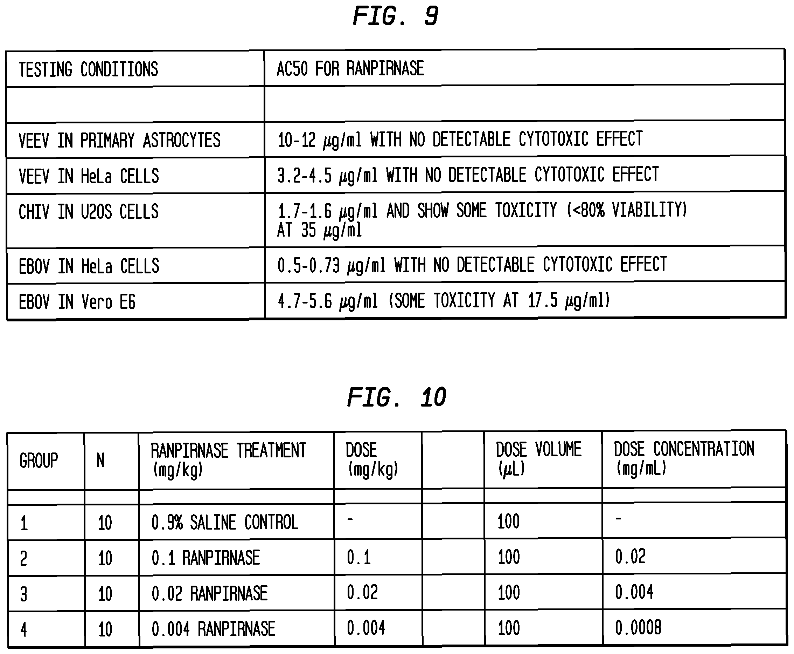

The results of the study showed that ranpirnase exhibited robust inhibition of VEEV, CHIV, and EBOV, with surprisingly low AC50 values and surprisingly high SI values. Figure 9 provides an overall summary of the AC50 results of the studies.

These experiments demonstrate that ranpirnase inhibited replication of the tested VEEV, CHIV, and EBOV in various mammalian cells (astrocytes, HeLa cells, U2OS cells, Vero E6 cells) without killing the cells themselves. These experiments further evidence the likelihood that systemically administered ranpirnase will be useful in treating a mammalian subject infected with a virus, and particularly a mammalian subject infected with VEEV, CHIV, and EBOV. Furthermore, it is to be noted that in these experiments, the ranpirnase was used prophylactically, in that the viruses were introduced into cells that had already been treated with ranpirnase. These experiments therefore constitute evidence that the antiviral qualities of ranpirnase can be used prophylactically as well as therapeutically.

### Comparative Example 5: EBOV in Mice

The effect of a range of doses of ranpirnase in EBOV infected mice was studied. C57BL/6 mice (eight to twelve weeks old) were infected with 1000 PFU of mouse-adapted EBOV-Zaire. Infection was accomplished by intraperitoneal injection (intravenous tail vain infusion). 1 hour before infection, each mouse received 0.9% saline (control group; Group 1),0.1 mg/kg ranpirnase (Group 2), 0.02 mg/kg ranpirnase (Group 3), or 0.004 mg/kg ranpirnase (Group 4). The study groups are shown in Figure 10.

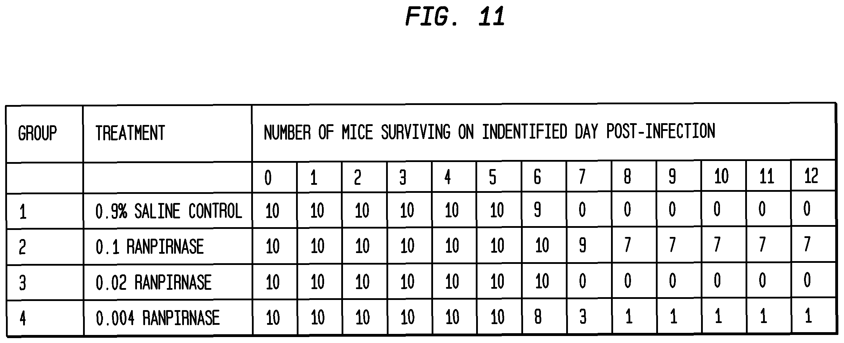

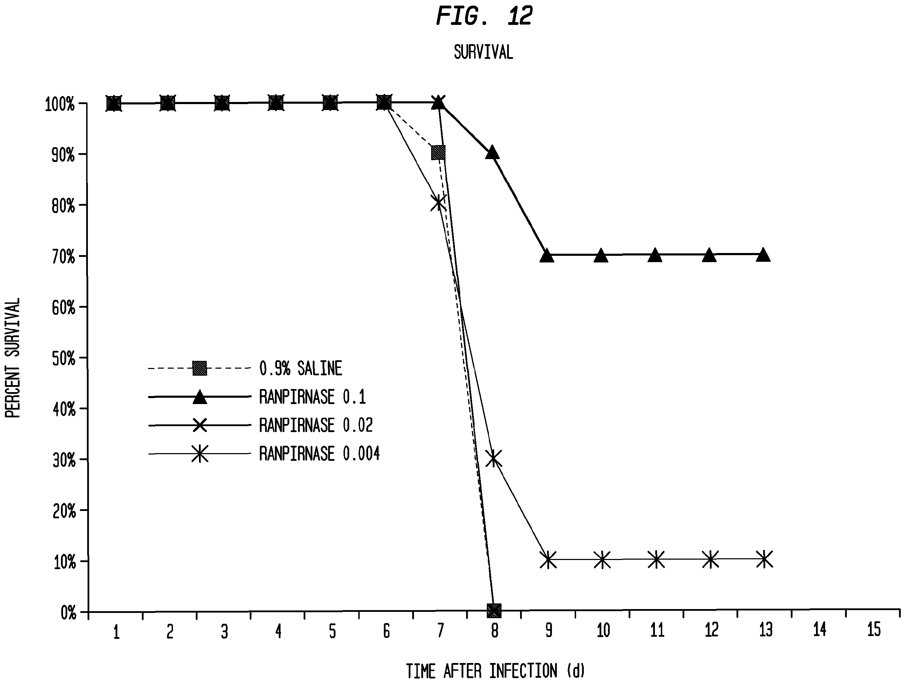

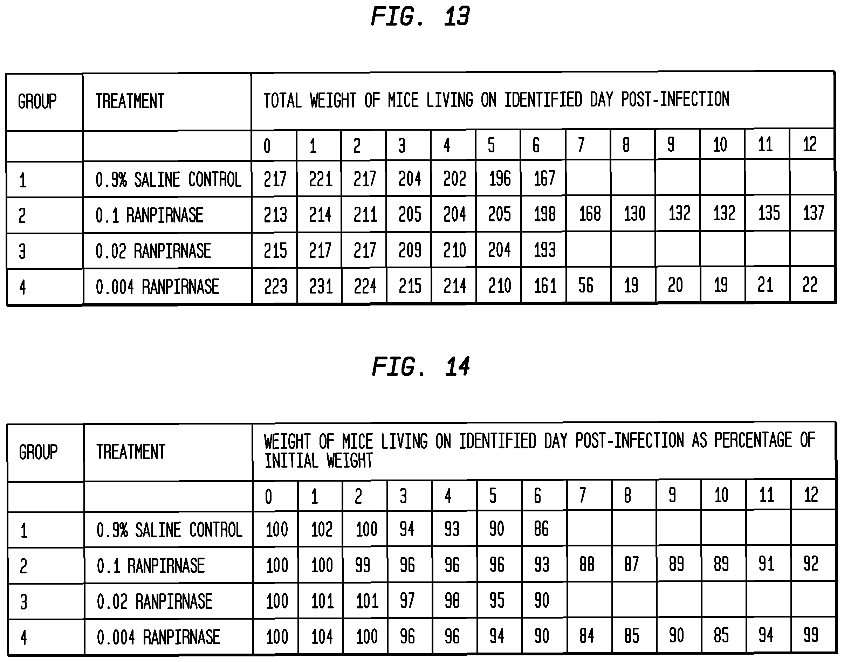

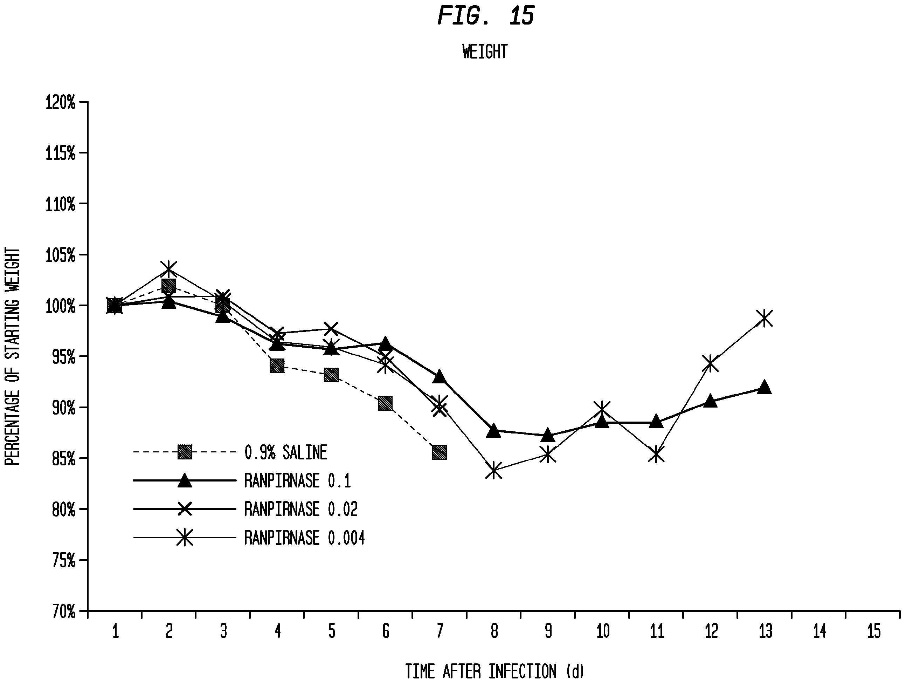

Mice were monitored for survival, and the weight of each mouse was obtained on the day of infection and every day for 14 days post infection. (Figures 11 and 12 show data for 12 days post infection, but monitoring of the mice continued for two additional days.) The number of survivors per group (out of 10 mice per group) and the percent survival in each group are provided in Figure 11 and Figure 12, respectively. The weight of the mice in each group and the percent change from the starting weight are shown below in Figures 13 and 14, respectively. The data in Figure 14 are also shown in graphical form in Figure 15.

The study showed that ranpirnase provides protection from lethal Ebola virus infection in mice. In particular, at a dose of 0.1 mg/kg ranpirnase, 70% of mice survived for at least 14 days after infection. In comparison, in control mice that did not receive ranpirnase, Ebola virus infection was 100% lethal by 7 days post-infection. In addition, surviving mice in the 0.1 mg/kg ranpirnase group maintained a weight within 92% of their starting weight at 12 days post-infection. Thus, the study showed that ranpirnase effectively promotes survival in Ebola virus infected mice. This still further evidences the likelihood that systemically administered ranpirnase will be useful in treating a mammalian subject infected with a virus, and particularly a mammalian subject infected with Ebola virus. Furthermore, the above-described experimental results in VEEV, CHIV, and EBOV indicate that it is possible to use ranpirnase as a prophylactic to prevent EBOV infection.

### Comparative Example 6: AV in NHBE Cells

A study was conducted to evaluate the anti-adenoviral (AV) activity of ranpirnase in NHBE cells.

Ranpirnase stock solution was prepared at 200µM and stored in aliquots at -80 °C. On the first day of the experiment, one aliquot was thawed and working solutions were prepared by appropriately diluting the stock concentration in cell culture medium. Working solutions were pre-warmed in a water bath set to 37 °C for 15 - 30 minutes prior to use.

Differentiated NHBE cells (MatTek Corporation, Ashland, MA) were used in the study. Cells were provided in kits with 12 or 24 tissue inserts each. The kits used in the study (EpiAirwayTM, AIR-110, and AIR-100, respectively) originated from a single human donor, # 9831, a 23-year old, healthy, non-smoking, Caucasian male. Upon arrival, tissue inserts were immediately transferred to individual wells of a 6-well plate according to manufacturer's instructions. Tissues were supplied with 1 ml of MatTek's culture medium (AIR-100-MM/Maintenance Medium) to the basolateral side, whereas the apical side was exposed to a humidified 95% air/5% CO₂ environment. Cells were equilibrated at 37 °C for at least 16 hours before the start of the experiment. After the equilibration period, the mucin layer, secreted from the apical side of the cells, was removed by repeated (3x) careful washing with 500 µl pre-warmed 30mM HEPES Buffered Saline Solution (Lonza, CC-5024 / Lot 0000354165) and the culture medium was replenished.

The virus (adenovirus 2, strain Miller from ATCC; titer 6.7 log10 CCID_{50/}0.1 ml) was stored at -80 °C prior to use. The dose level of adenovirus corresponded to an MOI of 0.1.

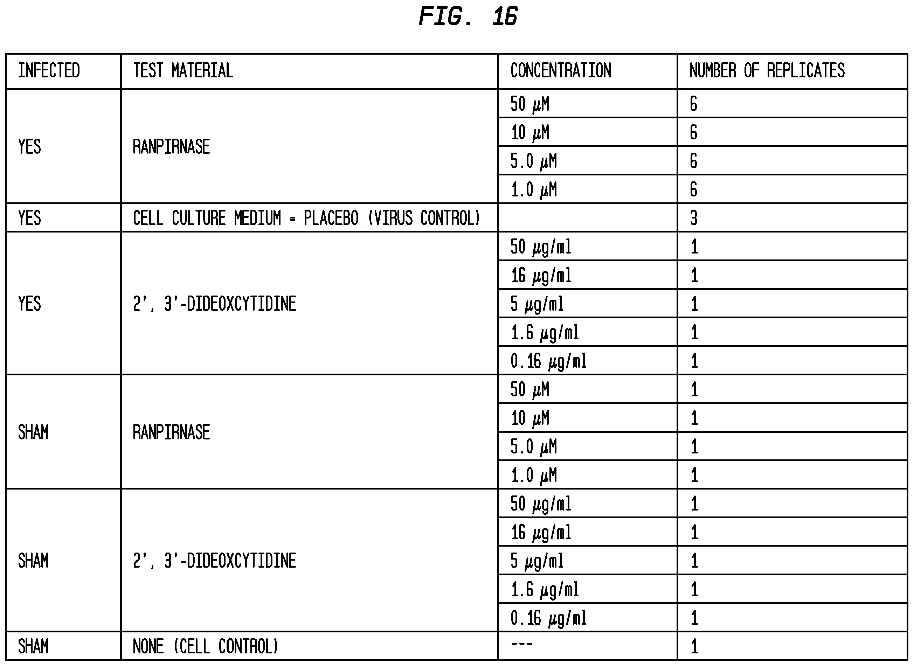

Differentiated NHBE cells were experimentally infected with the virus. After an adsorption period of 1 hour, the viral inoculum was removed and treatments were applied as shown in Figure 16. Twenty-four hours post infection, treatments were replenished in the basal compartment of the tissue inserts. Five days post infection, supernatants were harvested and stored at -80 °C until determination of virus titers in A-549 cells (human lung carcinoma cells from ATCC). Controls included four groups:
Group 1 - infected and placebo-treated cells (virus control);
Group 2 - sham-infected and treated cells (toxicity controls);
Group 3 - sham-infected and placebo-treated cells (cell control); and
Group 4 - 2',3'-dideoxcytidine as a positive control drug.
Toxicity controls were microscopically examined for possible changes in tissue and/or cell morphology at the end of the experiment.

NHBE cells were inoculated by exposure of the apical side to AV or cell culture medium (sham) as shown in Figure 16. After 1 hour ± 10 min of incubation at 37 °C and 5% CO₂, the viral inoculum or cell culture medium was removed from the cells. The apical side of the cells was washed once with 500 µl pre-warmed HEPES Buffered Saline Solution.

After inoculation, ranpirnase, 2',3'-dideoxcytidine, or cell culture medium (placebo/cell control) was added to the apical side of the cells and in the basal medium compartment, and incubated with the cells for 1 hour. After 1-hour incubation, the drug-containing medium was removed from the apical and basal chambers. Culture medium alone (placebo/cell control) or with drug (test condition) was added to the bottom chamber, and cells were incubated for 4 days. Twenty-four hours post infection, cell culture medium with and without drug was replenished to the basal compartment.

Following infection and treatment, cells were maintained at the air-liquid interface, and cell culture supernatant was harvested 48 hours post infection. Virus released into the apical compartment of the NHBE cells was harvested by the addition and collection of 500 µl culture medium allowed to equilibrate for 30 min at 37 °C and 5% CO₂. The medium from the apical compartment was divided into 2 aliquots, which were stored at -80 °C for future analysis of viral titers.

To assess the virus dose that was able to infect 50% of the cell cultures (CCID₅₀), A-549 cells were seeded in 96-well plates and grown overnight to achieve confluence, then washed twice with 100 µ1 infection medium (DMEM/EBSS supplemented with 50 µl/ml gentamycin). Wells were filled with 100 µl infection medium. Apical washes from the NHBE cell cultures were diluted 10-fold in infection medium and 100 µl were transferred into respective wells of a 96-well microtiter plate. Each concentration of ranpirnase from the NHBE cells (6 NHBE cell wells/dose) was titered leading to six titers per concentration (each NHBE well treated as a replicate) to evaluate the virus yields from infected and infected, treated cells. Thus, each concentration of ranpirnase was titered a total of six times. For the positive control, 2'3'-dideoxycytidine, one well of NHBE cells only was assigned to each concentration. Thus, each concentration was titered only once. Three wells were assigned as untreated, infected controls. They were titered once, resulting in three replicate untreated, infected control titers. After 6 days of incubation at 37 °C and 5% CO₂, cells were microscopically examined and scored for virus-induced cytopathic effect ("CPE"). A well was scored positive if any trace of CPE (usually cell rounding or lysis) was observed as compared with the uninfected control. CCID₅₀ was calculated by the Reed-Muench method.

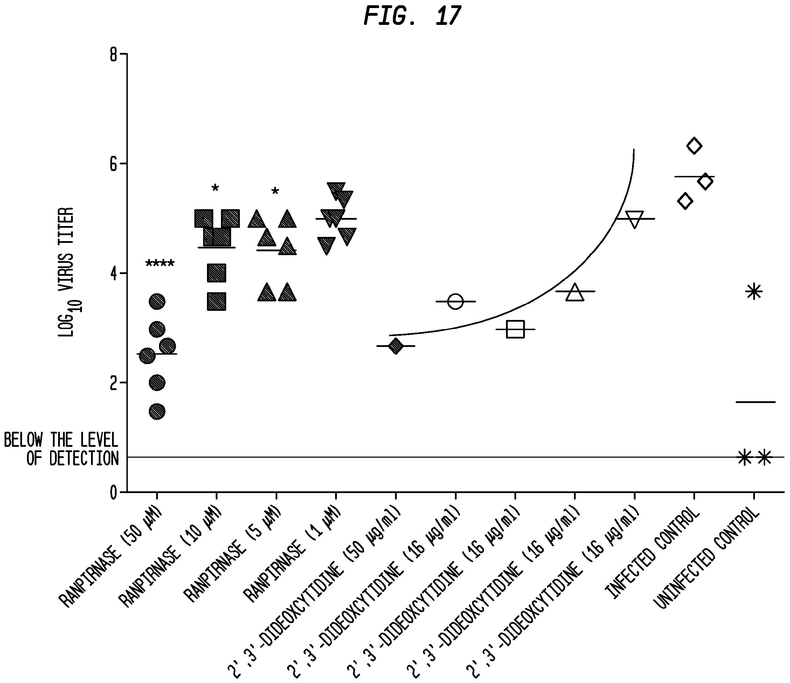

The results of the study are provided in Figure 17. All treatments with ranpirnase decreased virus titers to undetectable levels, except for the lowest dose. This reduction represented an approximately three log drop in virus titer for the 50 µM ranpirnase treatment (four asterisks indicates that p<0.0001 as compared to placebo). For the 5 and 10 µM ranpirnase treatments, a ∼1 log reduction in virus titers was detected when compared to the virus titers detected from the untreated, infected controls wells (a single asterisk indicates that p<0.05 as compared to placebo). 2'3'-dideoxycytidine inhibited virus replication as expected.

No virus cytopathic effects were detected in uninfected, ranpirnase-treated or 2'3'-dideoxycytidine-treated cells. Microscopy evaluations of ranpirnase-treated or 2'3'-dideoxycytidine-treated NHBE cells revealed no toxicological phenomena.

Therefore, the results of the study showed that all higher doses of ranpirnase treatment reduced AV titers in a statistically significant manner. Because ranpirnase was effective at inhibiting replication of AV in NHBE cells while not killing the host cells, this experiment further evidences the likelihood that systemically administered ranpirnase will be useful in treating a mammalian subject infected with a virus, and particularly a mammalian subject infected with AV. Furthermore, the above-disclosed experimental results in VEEV, CHIV, and EBOV indicate that it should be possible to use ranpirnase as a prophylactic to prevent AV infection.

The above data were acquired using adenovirus-2, which is a member of the adenoviridae family of viruses. The viruses in this family are very closely related and the demonstrated antiviral activity of ranpirnase against any one virus within the adenoviridae family is strong evidence that ranpirnase will have the same anti-replication activity against all viruses within the adenoviridae family.

### Comparative Example 7: Canine parvovirus in A-72 Cells

Canine parvovirus type 2c was grown in A-72 cell cultures and virus contained in cell culture medium (Dulbecco's minimum essential medium; "DMEM") was stored frozen at -80°C until used. The titer of the virus stock was about 105 cell culture infectious dose units/ml, with a hemagglutination (HA) titer of 1,024 to 2,048.

The A-72 continuous cell line, a fibroblastic line derived from a canine tumor, was grown in DMEM in 96-well plates, allowing for various conditions to be tested.

To assess the cytotoxicity of ranpirnase in A-72 cells, A-72 cells were seeded into a 24-well plate at a concentration of 125,000 cells/well in a volume of 1 ml of DMEM. The medium in each well was supplemented with 25µl of ranpirnase to concentration of 5µg/ml, 1 µg/ml, 0.5 µg/ml and 0.1 µg/ml. Each concentration was tested in 4 replicates. After incubating at 35.5°C for 4 days, the cells were examined under an inverted microscope for any visual morphologic changes.

Next, the efficacy of ranpirnase was assessed. Ranpirnase was tested at various concentrations ranging from 10 µg/ml down to 0.15625 µg/ml in 2-fold dilutions. Stock virus was tested from undiluted through a dilution of 1/10,000. A-72 cells were trypsinized following standard procedures and seeded into 96-well cell culture plates at a concentration of 18,750 cells/well in a volume of 150 µl. Ranpirnase was then added to each well to attain a final pre-determined concentration. After incubating for 2 hours at 35.5°C, 25 µl of virus at various dilutions was added. Controls included ranpirnase-treated/uninfected cells and ranpirnase inhibition of canine parvovirus replication in cell cultures of untreated/uninfected cells. All variables were tested in duplicate. At the end of 4 days of incubation at 35.5°C, the supernatant fluid was harvested and tested for evidence of virus replication using the HA test (which tests the ability of the virus to agglutinate a 0.5% suspension of swine red blood cells). HA titers were expressed as the reciprocal of the highest dilution of supernatant fluid that induced visual agglutination of the red blood cells in a 96-well plate.

When ranpirnase-treated (various concentrations) and untreated cells were microscopically examined, there was no visual difference in morphology between the two groups, regardless of ranpirnase concentration. Thus, there was no apparent cytotoxic effect of ranpirnase at any of the concentrations tested.

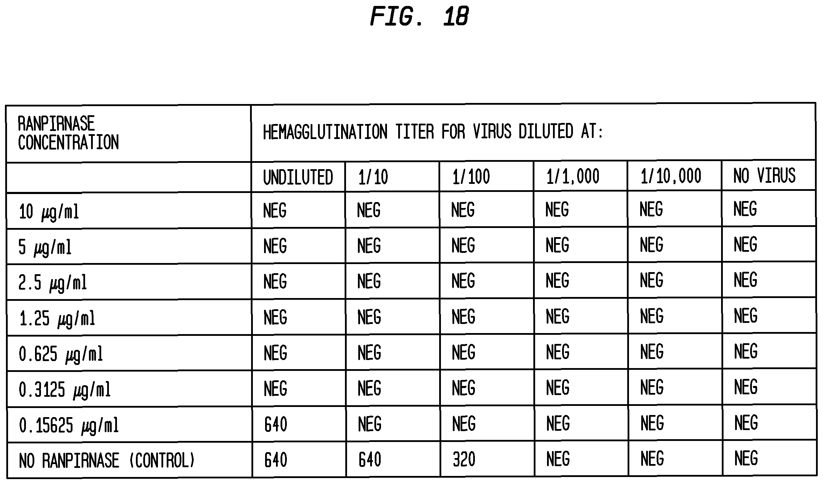

The results of the study are provided in Figure 18. Virus growth was detected in untreated cell cultures up to a virus dilution of 1/100. Virus growth was also detected when the undiluted virus stock was used to infect cell cultures treated with 0.15625 µg/ml ranpirnase. There was no virus growth in cultures treated with a ranpirnase concentration of 0.3125 µg/ml or higher. Because ranpirnase was so effective at inhibiting viral growth of canine parvovirus while not killing the host cells, this experiment further evidences the likelihood that systemically administered ranpirnase will be useful in treating a mammalian subject infected with a virus, and particularly a mammalian subject infected with canine parvovirus. Furthermore, the above-disclosed experimental results in VEEV, CHIV, and EBOV indicate that it should be possible to use ranpirnase as a prophylactic to prevent canine parvovirus infection.

### Comparative Example 8: RSV in NHBE Cells

Ranpirnase stock solution was prepared, stored, thawed, and used to prepare working solutions as described in Example 6.

NHBE cells from MatTek Corporation were used in the study. They were the same cell line as was used in Example 6 and were provided in the same kits. As in the Example 6, tissue inserts were immediately transferred to individual wells of a 6-well plate according to manufacturer's instructions. Tissues were supplied with 1 ml of the same culture medium used in Example 6 to the basolateral side, and the apical side was exposed to a humidified 95% air/5% CO₂ environment. Cells were equilibrated as in Example 6, and after this equilibration period, the mucin layer was removed as in Example 6 and the culture medium was replenished.

The virus (RSV A2 from ATCC; titer 4.7 log10 CCID₅₀/0-1 ml) was stored at - 80 °C prior to use. The dose level of challenge virus corresponds to an MOI of 0.1.

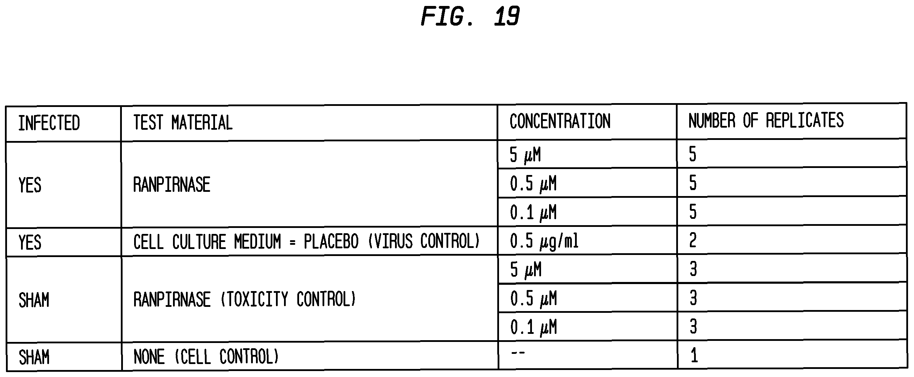

Differentiated NHBE cells were experimentally infected with the RSV virus. After an adsorption period of 1 hour, the viral inoculum was removed and treatments were applied as shown in Figure 19. Twenty-four hours post infection, treatments were replenished in the basal compartment of the tissue inserts. Forty-eight hours post infection, supernatants were harvested and stored at -80 °C until determination of virus titers in MA-104 cells (embryonic African green monkey kidney cells from ATCC). Controls consisted of three of the four groups used in the AV experiment:
Group 1 - infected and placebo-treated cells (virus control);
Group 2 - sham-infected and treated cells (toxicity controls); and
Group 3 - sham-infected and placebo-treated cells (cell control).
Toxicity controls were microscopically examined for possible changes in tissue and/or cell morphology at the end of the experiment.

NHBE cells were inoculated by exposure of the apical side to RSV or cell culture medium (sham) as shown in Figure 19. After 1 hour ± 10 min of incubation at 37 °C and 5% CO₂, the viral inoculum or cell culture medium was removed from the cells. The apical side of the cells was washed once with 500 µl pre-warmed HEPES Buffered Saline Solution.

After inoculation, ranpirnase, or cell culture medium (placebo/cell control) was added to the apical side of the cells and in the basal medium compartment, and incubated with the cells for 1 hour. After 1 -hour incubation, the drug-containing medium was removed from the apical and basal chambers. Culture medium alone (placebo/cell control) or with drug (test condition) was added to the bottom chamber, and cells were incubated for an additional 23 hours (24 hour total post-infection incubation). Twenty-four hours post infection, cell culture medium with and without drug was replenished to the basal compartment.

Following infection and treatment, cells were maintained at the air-liquid interface, and cell culture supernatant was harvested 48 hours ± 30 min post infection. Virus released into the apical compartment of the NHBE cells was harvested by the addition and collection of 500 µl culture medium allowed to equilibrate for 30 min at 37 °C and 5% CO₂. The medium from the apical compartment was divided into 2 aliquots, which were stored at -80°C for future analysis of viral titers. Images of one replicate tissue insert per treatment were taken with an inverted microscope at 40 and 100x, respectively, prior to harvest.

To assess the CCID₅₀, MA-104 cells were seeded in 96-well plates and grown overnight to achieve confluence, then washed twice with 100 µl infection medium (MEM/EBSS supplemented with 50 µl/ml gentamycin). Wells were filled with 100 µl infection medium. Apical washes were diluted 10-fold in infection medium and 100 µl were transferred into respective wells of a 96-well microtiter plate. Each sample was titered in triplicate (Passage 1) to evaluate the virus yields from infected and infected, treated cells. After 6 days of incubation at 37 °C and 5% CO₂, cells were microscopically examined and scored for virus-induced cytopathic effect (CPE). A well was scored positive if any trace of cytopathic effect (usually cell rounding or syncytium) was observed as compared with the uninfected control. CCID50 was calculated by the Reed-Muench method.

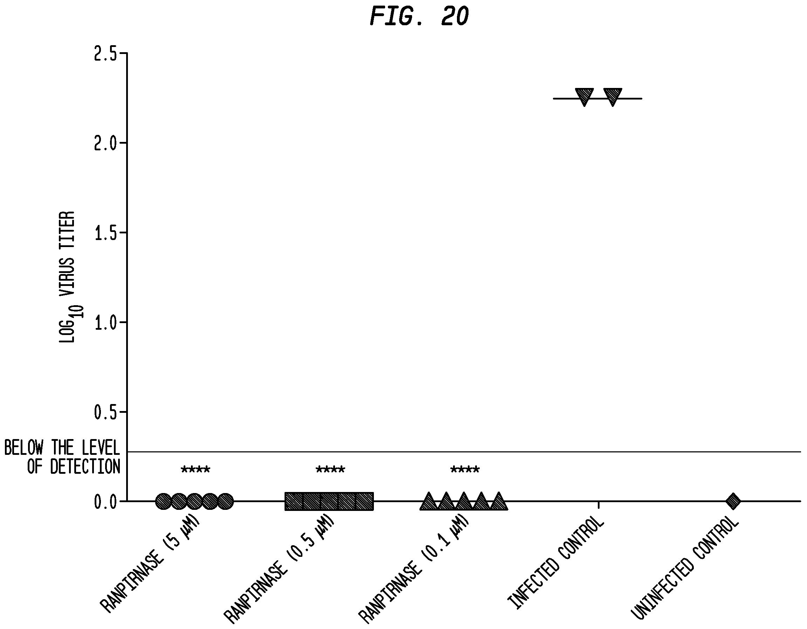

The results of the study are provided in Figure 20. Results were analyzed for statistical significance by one-way ANOVA (GraphPad Prism, version 6c). All treatments with Ranpirnase decreased virus titers to undetectable levels (Figure 20). This reduction represented a 2.3 log reduction in virus titers compared to untreated, infected controls (the four asterisks shown in Figure 20 indicate that p < 0.0001).

No virus cytopathic effects were detected Ranpirnase-treated/sham infected cells or uninfected control cells. Microscopy evaluations of sham infected, Ranpirnase-treated or 2'3'-dideoxycytidine-treated NHBE cells revealed no toxicological phenomena.

Therefore, the results of the study showed that all doses of ranpirnase tested (5 µM, 0.5 µM, and 0.1 µM) reduced RSV titers in a statistically significant manner, and that ranpirnase alone did not elicit cytopathic effects in NHBE cells. Because ranpirnase was so effective at inhibiting RSV while not killing the host cells, this experiment further evidences the likelihood that systemically administered ranpirnase will be useful in treating a mammalian subject infected with a virus, and particularly a mammalian subject infected with RSV. Furthermore, the above-disclosed experimental results in VEEV, CHIV, and EBOV indicate that it should be possible to use ranpirnase as a prophylactic to prevent RSV infection.

### Comparative Example 9: Rhinovirus-14 in NHBE Cells

Ranpirnase stock solution was prepared, stored, thawed, and used to prepare working solutions as described in Examples 6 and 8. NHBE from MatTek Corporation were used in the study. They were the same cell line as were used in Examples 6 and 8 and were provided in the same kits. As in Examples 6 and 8, tissue inserts were immediately transferred to individual wells of a 6-well plate according to manufacturer's instructions. Tissues were supplied with 1 ml of the same culture medium used in Examples 6 and 8 to the basolateral side, and the apical side was exposed to a humidified 95% air/5% CO₂ environment. Cells were equilibrated as in Example 6, and after this equilibration period, the mucin layer was removed as in Examples 6 and 8 and the culture medium was replenished.

RV-14 (strain 1059 from ATCC) was stored at -80°C prior to use. The titer of the stock virus was equal to titer 3.6 log10 CCID50/0.1 ml. The dose level of challenge virus was based on data from the previous experiments, and corresponded to a multiplicity of infection (MOI) of 0.0041.

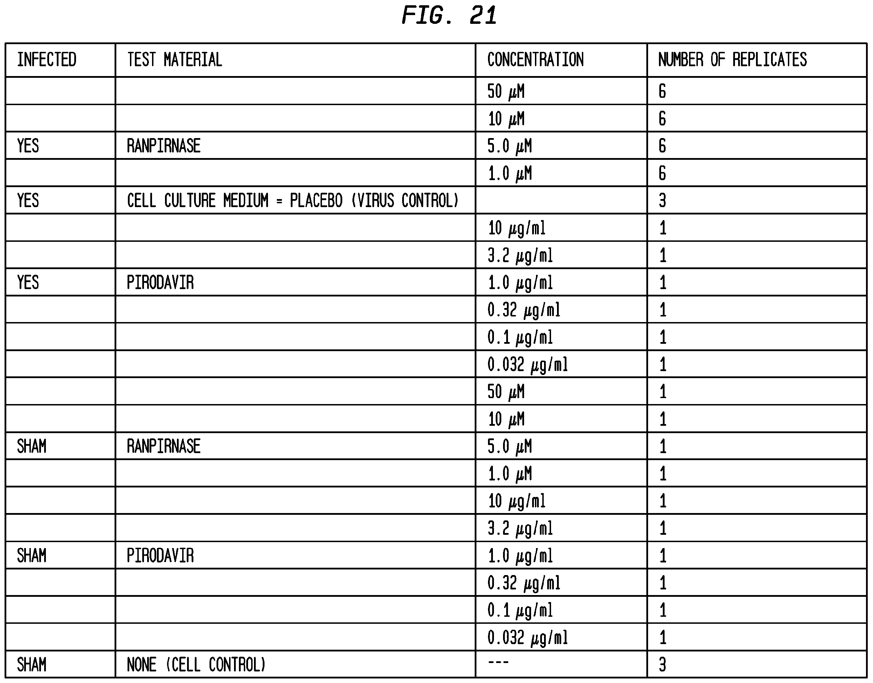

Differentiated NHBE cells were experimentally infected with RV-14 virus. After an adsorption period of 1 hour, the viral inoculum was removed and treatments applied (Figure 21). Twenty-four hours post infection, treatments were replenished in the basal compartment of the tissue inserts. Four days post infection, supernatants were harvested and stored at -80°C until determination of virus titers in HeLa-Ohio-1 cells (human cervical carcinoma cells from ATCC). Controls consisted of four groups:
Group 1 - infected and placebo-treated cells (virus control);
Group 2 - sham-infected and treated cells (toxicity controls);
Group 3 - sham-infected and placebo-treated cells (cell control); and
Group 4 - pirodavir as a positive control drug.

Toxicity controls were microscopically examined for possible changes in tissue and/or cell morphology at the end of the experiment.

NHBE cells were inoculated by exposure of the apical side to RV-14 or cell culture medium (sham infection) as seen in Figure 21. After 1 hour ± 10 min of incubation at 37 °C and 5% CO₂, the viral inoculum or cell culture medium was removed from the cells. The apical side of the cells was washed once with 500 µl pre-warmed HEPES Buffered Saline Solution.

After viral inoculation, ranpirnase, pirodavir, or cell culture medium (placebo/cell control) was added to the apical side of the cells and in the basal medium compartment, and incubated with the cells for 1 hour. After 1-hour incubation, the drug-containing medium was removed from the apical and basal chambers. Culture medium alone (Placebo/Cell control) or with drug (test condition) was added to the bottom chamber, and cells were incubated for 4 days. Twenty-four hours post infection, cell culture medium with and without drug was replenished to the basal compartment.

Following infection and treatment, cells were maintained at the air-liquid interface, and cell culture supernatant was harvested 4 days post virus exposure. Virus released into the apical compartment of the NHBE cells was harvested by the addition and collection of 500 µl culture medium allowed to equilibrate for 30 min at 37°C and 5%CO₂. The medium from the apical compartment divided into 2 aliquots, which were stored at -80°C for future analysis of viral titers.

HeLa Ohio-1 cells were seeded in 96-well plates and grown overnight to achieve confluence, then washed twice with 100 µl infection medium (MEM/EBSS supplemented with 50 µl/ml gentamycin). Wells were filled with 100 µl infection medium. Apical washes from the NHBE cell cultures were diluted 10-fold in infection medium and 100 µl were transferred into respective wells of a 96-well microtiter plate. Each concentration of ranpirnase from the NHBE cells (6 NHBE cell wells/dose) was titered leading to six titers per concentration (each NHBE well treated as a replicate) to evaluate the virus yields from infected and infected, treated cells. Thus, each concentration of Ranpirnase was titered a total of six times. For the positive control, pirodavir, one well of NHBE cells only was assigned to each concentration. Thus, each concentration was titered only once. Three wells were assigned as untreated, infected controls. They were titered once, resulting in three replicate untreated, infected control titers. After 7 days of incubation at 37°C and 5% CO₂, cells were microscopically examined and scored for virus-induced CPE. A well was scored positive if any trace of CPE (cell lysis) was observed as compared with the uninfected control. CCID50 was calculated by the Reed-Muench method and the inverse of that dilution represented the virus titer.

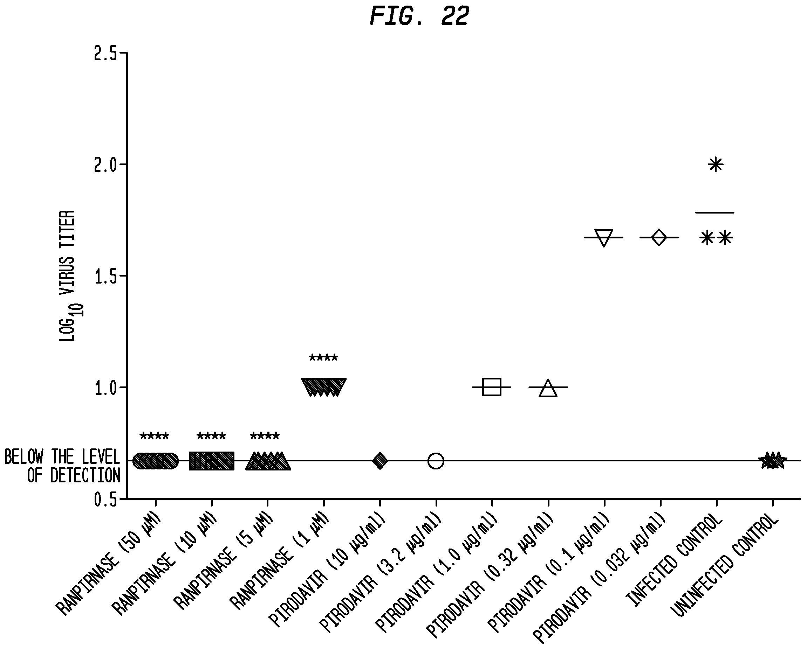

All ranpirnase treatments decreased virus titers relative to the titers of untreated infected controls except for the lowest dose (Figure 22). The reduction in virus titer with 50, 10, 5 µM ranpirnase treatment represented an approximate 1.67 log10 drop in virus titer for the (P<0.0001). For the 1 µM ranpirnase treatment, an ∼ 1 log reduction in virus titers was detected when compared to the virus titers detected from the untreated, infected controls wells (P<0.0001). Pirodavir inhibited virus replication as expected at 10 and 3.2 µg/ml with a somewhat dose responsive decrease in virus yields at subsequent lower dilutions of drug. Typically at 0.0032 µg/ml, pirodavir is also inactive against RV-14 in a HeLa Ohio-1 cell culture antiviral system as was seen in NHBE cells in this experiment.

No virus cytopathic effects were detected in uninfected, ranpirnase-treated or pirodavir-treated cells. Microscopy evaluations of ranpirnase-treated or pirodavir-treated NHBE cells revealed no toxicological phenomena.

Therefore, the results of the study showed that all doses of ranpirnase tested (50 µM, 1.0 µM, 5 µM and 1 µM) reduced RV-14 titers in a statistically significant manner, and that ranpirnase alone did not elicit cytopathic effects in NHBE cells. Because ranpirnase was so effective at inhibiting RV-14 while not killing the host cells, this experiment further evidences the likelihood that systemically administered ranpirnase will be useful in treating a mammalian subject infected with a virus, and particularly a mammalian subject infected with RV-14. Furthermore, the above-disclosed experimental results in VEEV, CHIV, and EBOV indicate that it should be possible to use ranpirnase as a prophylactic to prevent RV-14 infection.

These data were acquired using rhinovirus-14, an important member of the rhinovirus family of viruses. The viruses in this family are very closely related and the demonstrated antiviral activity of ranpirnase against any one virus within the rhinovirus family is strong evidence that ranpirnase will have the same anti-replication activity against all viruses within the rhinovirus family.

### Comparative Example 10: Vaccinia in Vero 76 Cells

Ranpirnase stock solution was prepared, stored, thawed, and used to prepare working solutions as described in Examples 6, 8 and 9.

The virus (vaccinia, strain WR from ATCC) was stored at -80°C prior to use. The titer of the stock virus was equal to titer 7.7 log10 CCID50/0.1 ml. The dose level of challenge virus was based on data from other experiments, and corresponded to 50-100 viral plaques/well.

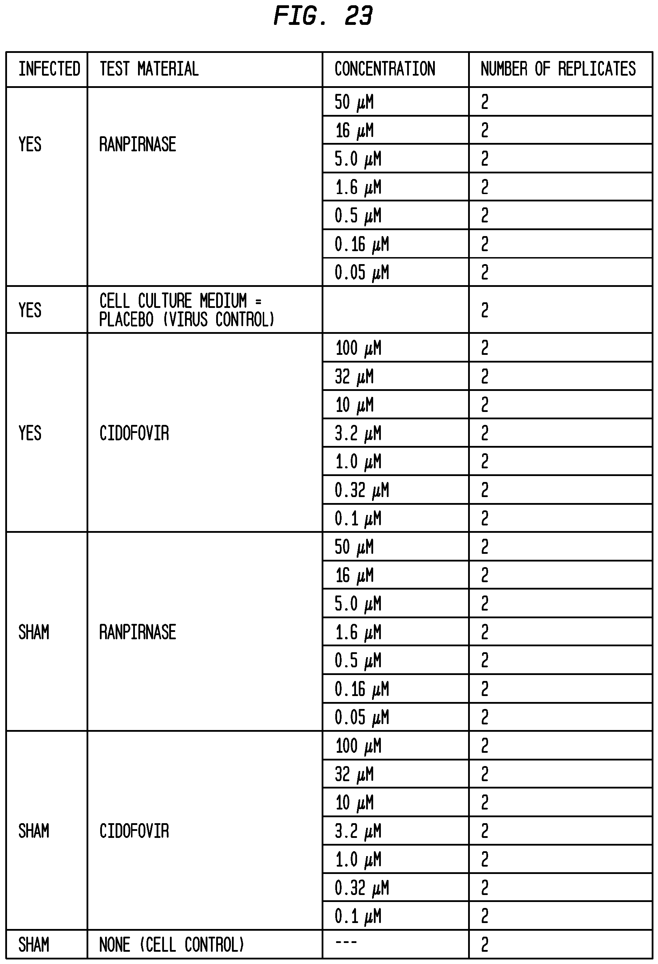

To determine the antiviral effect of ranpirnase against vaccinia, Vero 76 cells in 24 well plates were experimentally infected with virus in the format shown in Figure 23. Controls consisted of four groups:
Group 1 - infected and placebo-treated cells (virus control);
Group 2 - sham-infected and treated cells (toxicity controls);
Group 3 - sham-infected and placebo-treated cells (cell control); and
Group 4 - cidofovir as a positive control drug.

Toxicity controls were microscopically examined for possible changes in tissue and/or cell morphology at the end of the experiment and for staining intensity by crystal violet.

The assay was done in Vero 76 cells in 24-well plates as described above in Figure 23. The virus was pre-titrated to produce 50-100 viral plaques in 48 hours and absorbed to the cells for 1 hour at 37 °C. The virus inoculum was then removed and test compounds in agarose (described below) were added to the wells where appropriate.

Dilutions of Ranpirnase and cidofovir (the positive control) were made using a half-log10 dilution series (Figure 23). Two microwells were used per dilution. The medium for these assays contained 1% final concentration of agarose (Sea Plaque agarose from FMC Corp.) in 2% FBS in MEM. The agar overlay containing test compound, positive control compound, or no compound (virus control) was hardened in the refrigerator for 5-10 minutes prior to incubation at 37 °C. At 2 days post virus exposure, the wells of each plate were overlaid with 1 ml of 10% buffered formalin to fix the cells to the wells. Then the agar overlays were removed. The wells were stained with 0.1% crystal violet and the plaques were counted with the aid of a plaque viewer at either 13x magnification. The concentration of inhibitor reducing plaque numbers by 50% (EC50 value) was determined by plotting inhibitor concentration versus percentage of plaques. The determination of toxicity was done by examining the density of staining of the monolayers designated for toxicity evaluation. The less intense the staining compared to sham, untreated cells, the greater the toxicity. There was no toxicity detected by this procedure and thus it was unnecessary to calculate a CC50 value.

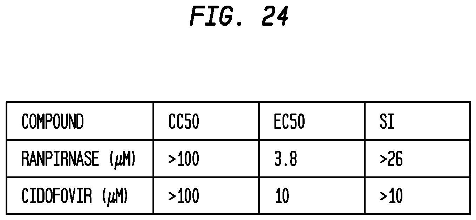

The antiviral effect of ranpirnase against vaccinia infection in Vero 76 cells was evaluated. Neither ranpirnase nor cidofovir was toxic at the dilutions used in the assay (Figure 24), which was in accordance with other previous studies in which toxicity for both drugs was quantified by neutral red uptake assay.

Ranpirnase strongly inhibited vaccinia replication at a good potent dose of 3.8 µM, which compared favorably with cidofovir (EC50 = 10 µM), the drug that is stockpiled by the Defense Department for use in case of a smallpox outbreak. Since ranpirnase was not toxic at 100 µM, it was highly selective in its inhibition of virus (SI >26).

Vaccinia is a member of the poxvirus family of viruses. The viruses in the poxvirus family are very closely related and the demonstrated antiviral activity of ranpirnase against any one virus within the poxvirus family is strong evidence that ranpirnase will have the same antiviral activity against all viruses classified within the poxvirus family (specifically including smallpox, which is such a serious biohazard that it cannot prudently be tested in the laboratory).

Furthermore, other above-disclosed experimental results in VEEV, CHIV, and EBOV indicate that it should be possible to use ranpirnase as a prophylactic to prevent vaccinia infection.

All the above data except those relating to VEEV and canine parvovirus are reasonably correlated with activity against viral infections in humans. These data constitute strong evidence that ranpirnase will be active against viruses in humans. The data relating to VEEV are reasonably correlated with activity against a VEEV infection in an equine species, and the data relating to canine parvovirus are reasonably correlated with activity against a canine parvovirus infection in dogs and other mammals.

Generally, in view of the different viruses that respond to treatment using ranpirnase, a person of ordinary skill in this art would conclude that any route by which ranpirnase is systemically administered will be adequate to treat any particular virus (although one route may be more effective than another in any particular instance). Thus, enteral administration (including without limitation oral administration and rectal administration) and parenteral administration (including without limitation intravenous administration, intramuscular administration, and aerosol delivery) are appropriate methods for administration of ranpirnase.

The above-recited experimental results were carried out using ranpirnase. However, other ribonucleases that are highly homologous to ranpirnase have exhibited highly similar activities against other viruses, such as herpesviridae viruses and human papillomavirus. These other ribonucleases include the RNases identified by SEQ ID Nos. 2, 3 and 4. To a person of ordinary skill in this art, the similarities of homology and activity of these three other ribonucleases is strong evidence that these three other ribonucleases will have the same activity as ranpirnase has. Hence, although the above-disclosed experiments have not yet been repeated using the '805 variant, Amphinase 2, or rAmphinase 2, it is believed that the above data are fully applicable to these three ribonucleases and that these three ribonucleases will be active against rabies, MERS-CoV, influenza, CHIV, EBOV, AV, RSV, RV, and poxvirus in humans, VEEV in equine species, and canine parvovirus in dogs and other mammals.

As demonstrated above, ranpirnase inhibits growth of MERS-CoV, VEEV, and CHIV, and RV-14 in various cell types. These three viruses are all categorized in Baltimore Classification Group IV. This is substantial evidence that systemically administered ranpirnase will be effective against viruses categorized in Baltimore Classification Group IV. And, based upon the similarities of homology and activity of the ' 805 variant, Amphinase 2, and rAmphinase 2 to the homology and activity of ranpirnase, these three other ribonucleases would be expected to have the same activity as ranpirnase against viruses classified in Baltimore Classification Group IV.

As demonstrated above, ranpirnase inhibits growth of influenza, Ebola, measles, RSV, and rabies in various cell types. These five viruses are all categorized in Baltimore Classification Group V. This is substantial evidence that systemically administered ranpirnase will be effective against viruses categorized in Baltimore Classification Group V. And, based upon the similarities of homology and activity of the ' 805 variant, Amphinase 2, and rAmphinase 2 to the homology and activity of ranpirnase, these three other ribonucleases would be expected to have the same activity as ranpirnase against viruses classified in Baltimore Classification Group V.

Although at least one preferred embodiment of the invention has been described above, this description is not limiting and is only exemplary. The scope of the invention is defined only by the claims, which follow:

### SEQUENCE LISTING

<110> Tamir Biotechnology, inc.
<120> RNase for use in treating or preventing viral infections
<130> 5027 PCT
<150> 14/229,816
   <151> 2014-03-28
<150> 14/247,723
   <151> 2014-04-08
<150> 14/316,893
   <151> 2014-06-27
<150> 14/667,282
   <151> 2015-03-24
<150> 62/040,885
   <151> 2014-08-22
<150> 62/063,551
   <151> 2014-10-14
<150> 62/102,671
   <151> 2015-01-13
<160> 4
<170> PatentIn version 3.5
<210> 1
   <211> 104
   <212> PRT
   <213> Rana pipiens
<400> 1
   Glu Asp Trp Leu Thr Phe Gln Lys Lys His Ile Thr Asn Thr Arg Asp
   1               5                   10                  15
   Val Asp Cys Asp Asn Ile Met Ser Thr Asn Leu Phe His Cys Lys Asp
               20                  25                  30
   Lys Asn Thr Phe Ile Tyr Ser Arg Pro Glu Pro Val Lys Ala Ile Cys
           35                  40                  45
   Lys Gly Ile Ile Ala Ser Lys Asn Val Leu Thr Thr Ser Glu Phe Tyr
       50                  55                  60
   Leu Ser Asp Cys Asn Val Thr Ser Arg Pro Cys Lys Tyr Lys Leu Lys
   65                  70                  75                  80
   Lys Ser Thr Asn Lys Phe Cys Val Thr Cys Glu Asn Gln Ala Pro Val
                   85                  90                  95
   His Phe Val Gly Val Gly Ser Cys
               100
<210> 2
   <211> 104
   <212> PRT
   <213> artificial
<220>
   <223> variant '805 of ranpirnase
<400> 2
   Glu Asp Trp Leu Thr Phe Gln Lys Lys His Val Thr Asn Thr Arg Asp
   1               5                   10                  15
   Val Asp Cys Asn Asn Ile Met Ser Thr Asn Leu Phe His Cys Lys Asp
               20                  25                  30
   Lys Asn Thr Phe Ile Tyr Ser Arg Pro Glu Pro Val Lys Ala Ile Cys
           35                  40                  45
   Lys Gly Ile Ile Ala Ser Lys Asn Val Leu Thr Thr Ser Glu Phe Tyr
       50                  55                  60
   Leu Ser Asp Cys Asn Val Thr Ser Arg Pro Cys Lys Tyr Lys Leu Lys
   65                  70                  75                  80
   Lys Ser Thr Asn Lys Phe Cys Val Thr Cys Glu Asn Gln Ala Pro Val
                   85                  90                  95
   His Phe Val Gly Val Gly Arg Cys
               100
<210> 3
   <211> 114
   <212> PRT
   <213> Rana pipiens
<400> 3 Lys Cys
<210> 4
   <211> 115
   <212> PRT
   <213> artificial
<220>
   <223> rAmphinase 2
<400> 4
   Met Lys Pro Lys Glu Asp Arg Glu Trp Glu Lys Phe Lys Thr Lys His
   1               5                   10                  15
   Ile Thr Ser Gln Ser Val Ala Asp Phe Asn Cys Asn Arg Thr Met Asn
               20                  25                  30
   Asp Pro Ala Tyr Thr Pro Asp Gly Gln Cys Lys Pro Ile Asn Thr Phe
           35                  40                  45
   Ile His Ser Thr Thr Gly Pro Val Lys Glu Ile Cys Arg Arg Ala Thr
       50                  55                  60
   Gly Arg Val Asn Lys Ser Ser Thr Gln Gln Phe Thr Leu Thr Thr Cys
   65                  70                  75                  80
   Lys Asn Pro Ile Arg Cys Lys Tyr Ser Gln Ser Asn Thr Thr Asn Phe
                   85                  90                  95
   Ile Cys Ile Thr Cys Arg Asp Asn Tyr Pro Val His Phe Val Lys Thr
               100                 105                 110
   Gly Lys Cys
           115

## Claims

1. RNase of the RNase A superfamily for use in treating or preventing viral infection in a mammalian subject, wherein the virus is classified in Baltimore Classification Group V, wherein the virus is from the orthomyxoviridae family and wherein the RNase is ranpirnase.

2. The RNase of the RNase A superfamily or functional derivative thereof for the use of claim 1, wherein the virus is influenza A.

3. The RNase of the RNase A superfamily or functional derivative thereof for the use of claim 1 or 2, wherein the mammalian subject is a human.

4. The RNase of the RNase A superfamily or functional derivative thereof for the use of any of claims 1 to 3, wherein the RNase is administered systemically.

## Patentansprüche

1. RNase aus der RNase A-Superfamilie zur Verwendung in der Behandlung oder Verhinderung einer viralen Infektion in einem Säugersubjekt, wobei das Virus in Baltimore-Klassifikationsgruppe V klassifiziert ist, wobei das Virus aus der Familie der Orthomyxoviridae stammt und die RNase Ranpirnase ist.

2. RNase aus der RNase A-Superfamilie oder funktionelles Derivat davon zur Verwendung nach Anspruch 1, wobei das Virus Influenza A ist.

3. RNase aus der RNase A-Superfamilie oder funktionelles Derivat davon zur Verwendung nach Anspruch 1 oder 2, wobei das Säugersubjekt ein Mensch ist.

4. RNase aus der RNase A-Superfamilie oder funktionelles Derivat davon zur Verwendung nach einem der Ansprüche 1 bis 3, wobei die RNase systemisch verabreicht wird.

## Revendications

1. RNase (ribonucléase) de la superfamille de la RNase A destinée à une utilisation dans le traitement ou dans la prévention d'une infection virale chez un sujet mammifère, dans laquelle le virus est classé dans le groupe V de la classification Baltimore, dans laquelle le virus est de la famille des orthomyxoviridae et dans laquelle la RNase est une ranpirnase.

2. RNase de la superfamille de la RNase A ou dérivé fonctionnel de celle-ci, destiné(e) à une utilisation selon la revendication 1, dans laquelle le virus est celui de l'influenza de type A.

3. RNase de la superfamille de la RNase A ou dérivé fonctionnel de celle-ci, destiné(e) à une utilisation selon la revendication 1 ou 2, dans laquelle le sujet mammifère est un humain.

4. RNase de la superfamille de la RNase A ou dérivé fonctionnel de celle-ci, destiné(e) à une utilisation selon l'une quelconque des revendications 1 à 3, dans laquelle la RNase est administrée de façon systémique.
